# EUROPEAN PATENT APPLICATION

(11) **EP 3 275 469 A1**
(43) Date of publication of application: **31.01.2018**
(21) Application number: 17183620.8
(22) Date of filing: 27.07.2017
(51) Int. Cl.: A61L 27/00

(54) **CARBON-BASED COMPOSITIONS USEFUL FOR OCCLUSIVE MEDICAL DEVICES AND METHODS OF MAKING AND USING THEM**

(30) Priority: 27.07.2016 US 201662367330 P
(71) Applicant: Contraline, Inc., Charlottesville, VA 22903 (US)
(72) Inventor: EISENFRATS, Kevin, Charlottesville VA 22902 (US); KAPADIA, Shrey, Centreville VA 20120 (US)
(74) Representative: Müller Fottner Steinecke

(57) **Abstract**

An occlusive device and a method of embolizing or occluding a bodily lumen by injecting or otherwise implanting the occlusive device are described. The device includes a polymer or polymer composition and a carbon-based material or nanomaterial such as graphene. The device may be used for sterilizing a human or animal by implanting the device into the vas deferens, fallopian tubes, or uterus, but may also be used to occlude any other bodily ducts, interstitial space, or organs.

## Description

### CROSS-REFERENCE TO RELATED APPLICATIONS

This application relies on the disclosure of and claims priority to and the benefit of the filing date of U.S. Provisional Application No. 62/367,330, filed on July 27, 2016, the disclosure of which is hereby incorporated by reference herein in its entirety.

### BACKGROUND OF THE INVENTION

### Field of the Invention

Embodiments of the present invention are directed to an occlusive device and a method of embolizing or occluding a body lumen by injecting or otherwise implanting the occlusive device, wherein the device comprises a polymer or polymer composition and acarbon-based material or nanomaterial, such as graphene. The device may be used for sterilizing a subject in need of contraception by implanting the device into the vas deferens, fallopian tube(s), or uterus, but may also be used to occlude any other body ducts, interstitial space, or organs.

### Description of the Related Art

One method for polymer implants in the body involves the embolization of blood vessels by blocking or sealing the vessel with a polymer hydrogel. This method has been shown to staunch bleeding and prevent blood clots, and it has proven moderately effective to prevent strokes and aneurysms. Another method taught by the prior art discloses embolizing the urethra to prevent urinary leakage in patients experiencing urinary incontinence disorder. Commercially, patients with a weak muscular urinary tract were aided by the injection of a polymer composition called URYX that stabilized the tract and in some cases prevented leakage.

Another method that has been explored in practice, but has never obtained regulatory approval for widespread application, is injecting or implanting a device into the vas deferens of the male. These contraceptives (e.g., RISUG, Vasalgel™, polyurethane, and silicone implants) could, in theory, be long-lasting and easily reversible, unlike vasectomy. However, most of the known techniques have met with instances of safety issues, lack of contraceptive efficacy, or inability to effectively reverse the implant, all of which are critical aspects for male contraception. RISUG and Vasalgel™, by way of popular example, are both vas-occlusive gels made of styrene maleic anhydride or styrene maleic acid, respectively *(see* U.S. Patent No. 5,488,075 and U.S. Patent Application Publication No. 20150136144).

Thus, an alternative polymer gel formulation or vas-occlusive device that is even more effective and reversible compared to vasectomy is missing from the reproductive and contraceptive field of medicine.

With respect to polymer composites containing carbon allotropes for biomedical applications, in one instance, an infrared and thermal responsive graphene oxide hydrogel is used as a drug delivery agent. The polymer is injected in a pill form and thermal or infrared radiation is used to make the polymer shrink and release the drug. The swelling ratio for the graphene-comprising hydrogel is at least 50% greater than the swelling ratio of the hydrogel polymer in the absence of graphene (*see* U.S. Patent No. 9,193,816). In another instance, graphene is a polymer hydrogel medicine carrier in which the graphene and polymer are created in an orally-administered pill form for controlled release within the body (*see* Chinese Patent Application No. CN203493942U). In another example, graphene is included in a hydrogel composition comprising a graphene, a chitosan, and a polyethylene (glycol)diacrylate (PEGDA). The polymer is similarly utilized in drug delivery and controlled release (*see* U.S. Patent Application Publication No. 20130230496). Another example of a graphene-based composition is described in Chinese Patent Application No. CN101812194B.

Graphene has also been incorporated into calcium silicate composites to study the effects of graphene on the composite's mechanical properties. The graphene composite showed improved fracture toughness by 130%, hardness by 30%, and brittleness index by 40% as compared to the CS matrix without GNP (*see* Mehrali, M., Moghaddam, E., Shirazi, S. F. S., Baradaran, S., Mehrali, M., Latibari, S. T. & Osman, N. A. A. (2014). Mechanical and in vitro biological performance of graphene nanoplatelets reinforced calcium silicate composite. PloS one, 9(9), e106802). Another study researched mechanical properties in polyethylene, such as fracture toughness, tensile strength, elastic modulus and yield strength, which were improved by adding graphene to high molecular weight polyethylene (*see* Lahiri, D., Dua, R., Zhang, C., de Socarraz-Novoa, I., Bhat, A., Ramaswamy, S., & Agarwal, A. (2012). Graphene nanoplatelet-induced strengthening of ultrahigh molecular weight polyethylene and biocompatibility in vitro. ACS applied materials & interfaces, 4(4), 2234-2241). Additionally, that same study showed a dose dependent effect on cytotoxicity of the graphene that could be attributed to agglomeration of the graphene at higher concentrations. The effect of graphene on the elastic modulus of chitosan composites has been shown to increase over 200% with the addition of 0.1%-0.3% graphene (*see* Fan, H., Wang, L., Zhao, K., Li, N., Shi, Z., Ge, Z., & Jin, Z. (2010). Fabrication, mechanical properties, and biocompatibility of graphene-reinforced chitosan composites. Biomacromolecules, 11(9), 2345-2351). The biocompatibility of graphene/chitosan composite films indicated that graphene/chitosan composites have acceptable biocompatibility.

While graphene has been incorporated into polymer and chitosan composites for biomedical applications in which the device must be taken orally, it appears graphene has not been previously taught for its ability to enhance the properties of occlusive devices. General efforts in this area include those described in U.S. Patent No. 9,193,816, US Patent Application Publication No. 20130230496, and Chinese Patent Nos. CN203493942U and CN101812194B, as described above. Yet, as with any art there remains a need for improvements. Furthermore, graphene has previously not been taught for its ability to be included in contraceptive medical devices to enhance the device's safety, efficacy, or durability.

### SUMMARY OF THE INVENTION

Embodiments of the invention provide compositions and methods for occluding a lumen of a vessel. According to embodiments, a carbon-based material or nanomaterial such as graphene is combined with a polymer to provide an occlusive medical device which has a variety of applications, including use as a male or female contraceptive device. These embodiments are based on the surprising discovery that carbon-based materials or nanomaterials such as graphene, may, at certain concentrations, enhance the contraceptive efficacy of occlusive devices by providing more favorable occlusive properties, spermicidal properties, viscosity, and/or life-span (via hardness and elasticity) while maintaining biocompatibility.

Included in embodiments of the invention is Aspect 1, which encompasses compositions comprising: a carbon-based nanomaterial or carbon allotrope, a polymer or network forming agent, and a solvent, wherein the carbon-based nanomaterial or carbon allotrope is present in the composition at a concentration, such as 10 ng/mL to 100 mg/mL in the end formulation, which enhances the efficacy of the composition as an occlusive agent upon administration into a body lumen.

Aspect 2 is the composition of Aspect 1, wherein the composition is a hydrogel or forms a hydrogel *in situ* upon administration into a body lumen.

Aspect 3 is the composition of Aspect 1 or 2, wherein the carbon-based nanomaterial or carbon allotrope is present in the composition at a concentration, such as 10 ng/mL to 100 mg/mL in the end formulation, which enhances the efficacy of the composition as a contraceptive agent.

Aspect 4 is the composition of any of Aspects 1-3, wherein the carbon-based nanomaterial or carbon allotrope is present in the composition at a concentration, such as 10 ng/mL to 100 mg/mL in the end formulation, that decreases the fertility, motility, and/or viability of sperm cells.

Aspect 5 is the composition of any of Aspects 1-4, wherein the carbon-based nanomaterial or carbon allotrope is present in the composition at a concentration, such as 10 ng/mL to 100 mg/mL in the end formulation, that increases the hardness and/or durability of the hydrogel.

Aspect 6 is the composition of any of Aspects 1-5, wherein the carbon-based nanomaterial or carbon allotrope is present in the composition at a concentration, such as 10 ng/mL to 100 mg/mL in the end formulation, that improves the mechanical properties of the hydrogel.

Aspect 7 is the composition of any of Aspects 1-6, wherein the carbon-based nanomaterial or carbon allotrope is present in the composition at a concentration, such as 10 ng/mL to 100 mg/mL in the end formulation, which alters the viscosity of the hydrogel.

Aspect 8 is the composition of any of Aspects 1-7, wherein the carbon-based nanomaterial or carbon allotrope is conjugated with a ligand comprising a small molecule, a protein, a peptide, an antibody, a nucleic acid, or fragment thereof, an aptamer, DNA, RNA, PNA, an enzyme, a sugar, a polysaccharide, a small molecule, a large molecule, a polymer, or a combination thereof.

Aspect 9 is the composition of any of Aspects 1-8, wherein the carbon-based nanomaterial or carbon allotrope conjugation is performed passively through adsorption with or without post-chemical activation of the carbon-based nanomaterial or carbon allotrope, actively through covalent bonding, or through placement of the ligand actively or passively to allow another molecule of interest to bind.

Aspect 10 is the composition of any of Aspects 1-9, wherein the carbon-based nanomaterial or carbon allotrope is functionalized with carboxylic acid (COOH) or a carboxylic group, amine (NH2), ammonia (NH3) or ammonium, pristine, argon (Ar), silicon (Si), a fluorocarbon, nitrogen (N2), fluorine (F), oxygen, alkyl, cycloalkyl, aryl, alkylaryl, amide, ester, ether, sulfonamide, carboxylate, sulfonate, phosphonate, fluorocarbons, carbonates, nitro, halogens (bromine, chlorine, fluorine), boron, boronic acids, biomacromolecules including sugars and proteins, a polymer, and supramolecular/coordination complexes including metal coordination complexes, and supramolecular complexes.

Aspect 11 is the composition of any of Aspects 1-10, wherein the carbon-based nanomaterial or carbon allotrope comprises one or more of graphene, graphene powder, graphene oxide, nanoscale graphene oxide, reduced graphene oxide, nanoscale graphene oxide, graphene nanoribbons, graphene nanotubes, graphene sheets, graphene films, granulated graphene, graphene quantum dots, graphene nanoribbons, graphene nanocoils, graphene aerogels, graphene nanoplatelets, carbon nanotubes, carbon nanosheets, carbon nanocones, carbon nanoribbons, buckyballs, and/or fullerenes.

Aspect 12 is the composition of any of Aspects 1-11, wherein the polymer comprises one or more of styrene maleic anhydride, styrene maleic acid, ethylene vinyl alcohol (EVOH), ethylene vinyl acetate, poly(ethylene glycol) (PEG), poly-L-lactic acid (PLLA), poly(lactic-co-glycolic acid) (PLGA), poly lactide (PLA), poly(glycolic acid) (PGA), poly(vinyl alcohol) (PVA), polydimethylsiloxane (PDMS), poly(isopropylacrylate) (PIPA), poly(ethylene-vinyl acetate) (PEVA), PEG styrene, any block copolymer, poly(styrene)-block-poly(ethylene glycol), a nylon polymer, Teflon RFE, polyetherketone etherketone ketone (PEKEKK), fluorinated high density polyethylene (FLPE), neoprene, (PETE), Teflon FEP, Teflon PFA, polymethylpentene (PMP), methyl palmitate, poly(N-isopropylacrylamide) (NIPA), polycarbonate, polyethersulfone, polycaprolactone, polymethyl methacrylate, polypropylene, polyurethane, polystyrene, polyisobutylene, nitrocellulose, medical grade silicon, cellulose acetate butyrate, polyacrylonitrile, poly(lactide-co-caprolactone) (PLCL), chitosan, alginate, polymethyl methacrylate, polyacrylonitrile, poly (carbonate-urethane), poly (vinylacetate), nitrocellulose, cellulose acetate, urethane, urethane/carbonate, polylactic acid, polyacrylamide (PAAM), poly (N-isopropylacrylamine) (PNIPAM), poly (vinylmethylether), poly (ethylene oxide), poly (ethyl (hydroxyethyl) cellulose), poly(2-ethyl oxazoline), poly(e-caprolactone), polydiaoxanone, polyanhydride, trimethylene carbonate, poly(β-hydroxybutyrate), poly(g-ethyl glutamate), poly(DTH-iminocarbonate), poly(bisphenol A iminocarbonate), poly(orthoester) (POE), polycyanoacrylate (PCA), polyphosphazene, polyethyleneoxide (PEO), polyacrylic acid (PAA), polyacrylonitrile (PAN), polyvinylpyrrolidone (PVP), polyglycolic lactic acid (PGLA), poly(2-hydroxypropyl methacrylamide) (pHPMAm), poly(vinyl alcohol) (PVOH), PEG diacrylate (PEGDA), poly(hydroxyethyl methacrylate) (pHEMA), N-isopropylacrylamide (NIPA), poly(vinyl alcohol) poly(acrylic acid) (PVOH-PAA), collagen, silk, fibrin, gelatin, hyaluron, cellulose, chitin, dextran, casein, albumin, ovalbumin, heparin sulfate, starch, agar, heparin, fibronectin, fibrin, keratin, pectin, and/or elastin.

Aspect 13 is the composition of any of Aspects 1-12, wherein the solvent comprises a buffered aqueous solution, dimethyl sulfoxide, ethyl acetate, methanol, acetone, acetonitrile, tetrahydrofuran (THF), dioxane, dimethylformamide (DMF), heptane, hexane, pentane, petroleum ether, benzene, toluene, o-xylene, m-xylene, p-xylene, ethanol, methanol, t-butyl alcohol, diethylene glycol, glycerol, ethylene glycol, chloroform, dichloromethane, 1,2-dichloroethane, hexafluoroisopropanol, a deuterated solvent, or a fluorocarbon solvent.

Aspect 14 is the composition of any of Aspects 1-13, wherein the hydrogel includes contrast agents, imaging agents, therapeutic drugs, antimicrobials, anti-inflammatories, spermicidal agents, vasodilators, steroids, hormones, ionic solutions, proteins, nucleic acids, antibodies, or fragments thereof.

Aspect 15 is a method of occluding a body lumen comprising: administering a composition into the body lumen, wherein the composition comprises a carbon-based nanomaterial or carbon allotrope, a polymer, and a solvent, and wherein the carbon-based nanomaterial or carbon allotrope is present in the composition at a concentration which enhances the efficacy of the composition as an occlusive agent upon administration into a body lumen; and polymerizing the composition or forming a mass from the composition in the body lumen. According to methods of embodiments of the invention, the composition administered can, for example, be any composition of any of Aspects 1-14, or combinations thereof.

Aspect 16 is the method of Aspect 15, wherein the body lumen is an artery, a vein, a vas deferens, a fallopian tube, a uterus, a duct, interstitial space, or an organ.

Aspect 17 is the method of Aspect 15 or 16, wherein the body lumen is a vas deferens or fallopian tube.

Aspect 18 is the composition of any of Aspects 1-14, wherein the carbon-based nanomaterial or carbon allotrope comprises graphene.

Aspect 19 is a method of contraception comprising administering the composition of any of Aspects 1-14 to a subject in a manner effective to provide contraception.

### BRIEF DESCRIPTION OF THE DRAWINGS

The accompanying drawings illustrate certain aspects of embodiments of the present invention and should not be used to limit the invention. Together with the written description the drawings serve to explain certain principles of the invention.
FIG. 1 is a graph showing the effect of styrene maleic acid with and without graphene nanoplatelets on sperm motility and viability.
FIGS. 2A and 2B are scanning electron microscopy images of a surface of an exemplary composition of the invention mixed by ultrasonication (FIG. 2A) and vortexing (FIG. 2B).
FIG. 3 is table showing measures of hardness and elasticity of a styrene maleic acid-graphene hydrogel exemplary composition of the invention.
FIG. 4 is a graph showing the effect of graphene nanoplatelets on the viscosity of a styrene maleic acid solution.
FIGS. 5A and 5B are photographs showing examples of electrospun polycaprolactone-graphene fiber (FIG. 5A) and mesh (FIG. 5B).

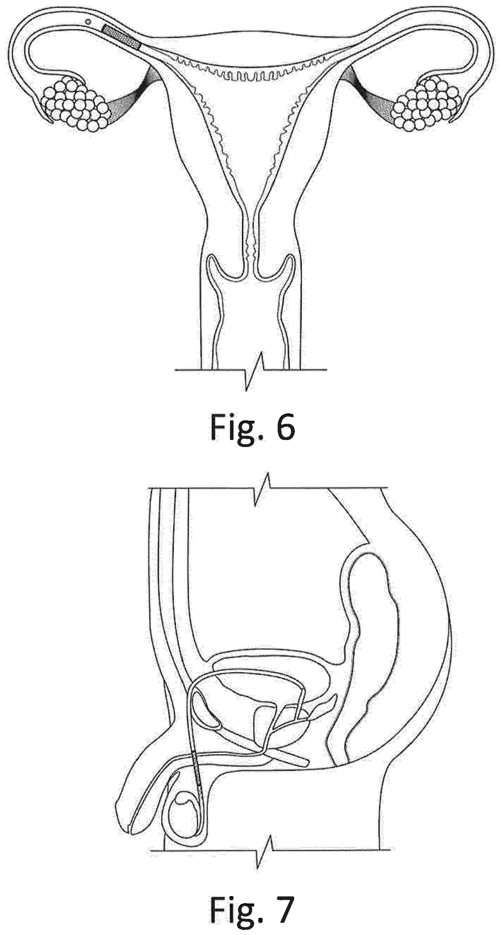

FIG. 6 is a schematic of a female reproductive system showing a device of an embodiment of the invention disposed in a fallopian tube.
FIG. 7 is a schematic of a male reproductive system showing a device of an embodiment of the invention disposed in a vas deferens.

### DETAILED DESCRIPTION OF THE INVENTION

Reference will now be made in detail to various exemplary embodiments of the invention. It is to be understood that the following discussion of exemplary embodiments is not intended as a limitation on the invention. Rather, the following discussion is provided to give the reader a more detailed understanding of certain aspects and features of the invention.

As used herein, a "carbon-based material" is any allotrope of carbon which includes is but not limited to diamond, graphite, graphene, fullerenes, amorphous carbon, single-walled carbon nanotubes, and multi-walled carbon nanotubes; for example.

As used herein, the term "solvent" means its art-recognized definition of a substance such as a liquid, a solid, a gas, or a supercritical fluid, that is capable of dissolving a substance.

As used herein, a "subject" is an animal. Such animals include mammals, including companion animals such as a dog or cat, farm animals such as a horse or cow, laboratory animals such as a mouse or rat, as well as non-human primates and humans.

As used herein, a "subject in need of contraception" is a patient, animal, mammal, or human, who will benefit from the method of this invention.

The term "biocompatible," as used herein, refers to a material that is compatible with living tissue or a living system when implanted by having a reduced or nontoxic, non-injurious, or non-physiologically reactive effect and not causing immunological rejection in the host, such as for example avoiding chronic inflammation or not inducing foreign body giant cells in the host.

The term "about," as used herein, means approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 10%. In one aspect, the term "about" means plus or minus 20% of the numerical value of the number with which it is being used. Therefore, about 50% means in the range of 40%-60%. Numerical ranges recited herein by endpoints include all numbers and fractions subsumed within that range (e.g. 1 to 5 includes 1, 1.5, 2, 2.75, 3, 3.90,4, and 5). It is also to be understood that all numbers and fractions thereof are presumed to be modified by the term "about."

A "fragment" or "segment" is a portion of an amino acid sequence, comprising at least one amino acid, or a portion of a nucleic acid sequence comprising at least one nucleotide. The terms "fragment" and "segment" are used interchangeably herein.

As used herein, the term "fragment," as applied to a protein or peptide, can ordinarily be at least about 3-15 amino acids in length, at least about 15-25 amino acids, at least about 25-50 amino acids in length, at least about 50-75 amino acids in length, at least about 75-100 amino acids in length, and greater than 100 amino acids in length.

As used herein, the term "fragment" as applied to a nucleic acid, may ordinarily be at least about 20 nucleotides in length, typically, at least about 50 nucleotides, more typically, from about 50 to about 100 nucleotides, preferably, at least about 100 to about 200 nucleotides, even more preferably, at least about 200 nucleotides to about 300 nucleotides, yet even more preferably, at least about 300 to about 350, even more preferably, at least about 350 nucleotides to about 500 nucleotides, yet even more preferably, at least about 500 to about 600 nucleotides, even more preferably, at least about 600 nucleotides to about 620 nucleotides, yet even more preferably, at least about 620 to about 650 nucleotides, and most preferably, the nucleic acid fragment will be greater than about 650 nucleotides in length.

As used herein, the term "nucleic acid" encompasses RNA as well as single and double-stranded DNA and cDNA. Furthermore, the terms, "nucleic acid," "DNA," "RNA" and similar terms also include nucleic acid analogs, i.e., analogs having other than a phosphodiester backbone. For example, the so-called "peptide nucleic acids," which are known in the art and have peptide bonds instead of phosphodiester bonds in the backbone, are considered within the scope of the present invention. By "nucleic acid" is meant any nucleic acid, whether composed of deoxyribonucleosides or ribonucleosides, and whether composed of phosphodiester linkages or modified linkages such as phosphotriester, phosphoramidate, siloxane, carbonate, carboxymethylester, acetamidate, carbamate, thioether, bridged phosphoramidate, bridged methylene phosphonate, bridged phosphoramidate, bridged phosphoramidate, bridged methylene phosphonate, phosphorothioate, methylphosphonate, phosphorodithioate, bridged phosphorothioate or sulfone linkages, and combinations of such linkages. The term nucleic acid also specifically includes nucleic acids composed of bases other than the five biologically occurring bases (adenine, guanine, thymine, cytosine, and uracil). Conventional notation is used herein to describe polynucleotide sequences: the left-hand end of a single-stranded polynucleotide sequence is the 5'-end; the left-hand direction of a double-stranded polynucleotide sequence is referred to as the 5'-direction. The direction of 5' to 3' addition of nucleotides to nascent RNA transcripts is referred to as the transcription direction. The DNA strand having the same sequence as an mRNA is referred to as the "coding strand"; sequences on the DNA strand which are located 5' to a reference point on the DNA are referred to as "upstream sequences"; sequences on the DNA strand which are 3' to a reference point on the DNA are referred to as "downstream sequences."

The term "peptide" typically refers to short polypeptides.

A "polynucleotide" means a single strand or parallel and anti-parallel strands of a nucleic acid. Thus, a polynucleotide may be either a single-stranded or a double-stranded nucleic acid.

"Polypeptide" refers to a polymer composed of amino acid residues, related naturally occurring structural variants, and synthetic non-naturally occurring analogs thereof linked via peptide bonds, related naturally occurring structural variants, and synthetic non-naturally occurring analogs thereof.

As used herein, an "effective amount" or "therapeutically effective amount" means an amount sufficient to produce a selected effect, such as alleviating symptoms of a disease or disorder. In the context of administering compounds in the form of a combination, such as multiple compounds, the amount of each compound, when administered in combination with another compound(s), may be different from when that compound is administered alone. Thus, an effective amount of a combination of compounds refers collectively to the combination as a whole, although the actual amounts of each compound may vary. The term "more effective" means that the selected effect is alleviated to a greater extent by one treatment relative to the second treatment to which it is being compared.

As used herein, a "non-surgical" (or "non-surgically") aspect of a procedure is performed without surgery. "Non-surgical isolation" refers to any non-surgical technique which identifies an anatomical part through the intact skin or connective tissues such that it is positioned underneath the skin or connective tissue. Thus, "non-surgical isolation" does not involve the use of a scalpel to incise the skin or the use of sutures to close the skin after incision.

Embodiments of the present invention describe polymeric medical devices and/or compositions comprising carbon-based materials or nanomaterials that are allotropes of carbon which include, but are not limited to, graphene, graphene powder, graphene oxide, nanoscale graphene oxide, reduced graphene oxide, nanoscale graphene oxide, graphene nanoribbons, graphene nanotubes, graphene sheets, graphene films, granulated graphene, graphene quantum dots, graphene nanoribbons, graphene nanocoils, graphene aerogels, graphene nanoplatelets, or any other carbon-based material or nanomaterial including but not limited to carbon nanotubes (single walled, double walled, or multiwalled), nanosheets, nanocones, nanoribbons, buckyballs, fullerenes, and the like. In embodiments, these devices are delivered in a form which includes a polymer and carbon-based material or nanomaterial mixture suspended, dispersed, dissolved, or present in a solvent. The solution may be delivered into an environment such as the arteries, veins, vas deferens, fallopian tubes, uterus, ducts, interstitial space, or organs in the body, via various methods including, but not limited to, in situ gelling, cross-linking, or precipitation, implantation of a hydrogel plug, insertion of electrospun fibers or mesh, or combinations thereof.

The gel, mesh, composition, or device occludes the tube, duct, or organ by preventing the flow of bodily fluid, cells, or other material. The gel, mesh, composition, or device can also act as a semi-permeable membrane, blocking some cells, fluid, or other material, while allowing other cells, material, and/or fluid to flow through.

One such application for the gel, mesh, composition, or device (herein referred to interchangeably as a gel, mesh, composition, device, occlusive device, occlusive composition, occlusive substance, or any other applicable definition of gel, mesh, composition, device, formulation, or other object or article) is the implantation of the polymer containing gel or mesh into the vas deferens or fallopian tubes for male and female contraception, respectively. The gel or mesh blocks sperm or the oocyte from traveling through the relevant tube(s), duct(s), and/or organ(s), thus causing temporary or permanent infertility; preferably, temporary infertility because the gel or mesh implantation can be reversed. The carbon-based material or nanomaterial, in addition to the physical and chemical properties of the polymeric gel or mesh, can also render sperm preferably nonviable or immotile through several mechanisms. Not wishing to be bound by theory, the material's sharp edges and high motility can allow for penetration of the carbon sheets into the spermatozoa to interact with cell nuclei (*see* Hashemi, E., Akhavan, 0., Shamsara, M., Rahighi, R., Esfandiar, A., & Tayefeh, A.R. (2014). Cyto and genotoxicities of graphene oxide and reduced graphene oxide sheets on spermatozoa. RSC Advances, 4(52), 27213-27223). Further, the morphology of the sperm may be affected due to physical and/or chemical interaction with the carbon-based nanomaterial such that the sperm heads are ruptured, or coiled or ripped off, and/or have a loss of proteins, carbohydrates, or other biomarkers that are important for fertilization. In embodiments, a carbon-based material or nanomaterial such as graphene is provided at concentrations which render it biocompatible, yet effective as part of a contraceptive device. By using an optimal concentration of graphene or other carbon-based material or nanomaterial, such as in the vas deferens, the graphene or other carbon-based material or nanomaterial can exhibit spermicidal properties that make the contraceptive device safe and efficacious. In embodiments, compositions of the invention comprising a carbon-based material or nanomaterial may reduce sperm motility, viability, and/or fertility in any combination. In embodiments, the compositions reduce the motility and viability below the World Health Organization's standards for viable and motile sperm, as ascertained by standard semen analysis tests known in the art. In embodiments, the carbon-based material or nanomaterial may reduce the total motility, progressive (or forward) motility, viability, fertility, and morphology of the sperm as assessed by such semen analysis tests. For example, embodiments of the compositions of the invention may, in an *in vitro* assay, render sperm with a viability below 60%, 55%, 50%, 45%, 40%, 35%, 30%, 25%, 20%, 15%, 10%, 5%, or lower, a total motility below 40%, 35%, 30%, 25%, 20%, 15%, 10%, 5%, or lower, and a progressive motility below 35%, 30%, 25%, 20%, 15%, 10%, 5%, or lower. In other embodiments, the compositions of the invention may reduce the morphology of the sperm such that less than 10%, 9%, 8%, 7%, 6%, 5%, 4%, 3%, 2%, 1%, or lower are normally shaped. Additionally, other spermicidal agents such as nonoxynol-9, oxtoxynol-9, benzalkonium chloride, or chlorhexidine can be included in the composition to enhance the spermicidal capability of the device. Additionally, the vas-occlusive polymer and/or solvent can be innately spermicidal, such that no exogenous spermicidal agent is included.

Embodiments of the compositions may also exhibit increased durability or lifespan through increased hardness, decreased elasticity, or both. In embodiments, the hardness of the compositions of the invention increases by at 10%, 15%, 20%, 25%, 30%, 35%, 40%, 45%, 50%, 55%, 60%, 65%, 70%, 75% or more with the addition of graphene or other carbon-based material or nanomaterial, while the elasticity decreases by 10%, 15%, 20%, 25%, 30%, 35%, 40%, 45%, 50%, 55%, 60%, 65%, 70%, 75% or more with the addition of the carbon-based material or nanomaterial. In embodiments, the durability or lifespan of the composition can increase such that the composition may substantially remain in the lumen for at least 0.5, 1, 1.5, 2. 2.5, 3, 3.5, 4.5, 5, 6, 7, 8, 9, 10, 15, 20, 25, 30, or 40 years or longer without degrading, such as when administered in a body lumen to occlude the lumen. In embodiments, the durability or lifespan of the composition can decrease such that the composition is made more degradable. For example, in embodiments, the composition may degrade, partially degrade, or substantially degrade after minutes or hour(s), such as 1 hour, 2 hours, 3 hours, 6 hours, 12 hours, 24 hours or 1 day, 2 days, 3 days, 7 days, 14 days, or 1 month, such as when administered in a body lumen to occlude the lumen.

The carbon-based material or nanomaterial portion of the device may be manufactured by any mechanical or chemical method known in the art. For example, the production of graphene includes, but is not limited to, ion implementation, microwave-assisted oxidation, supersonic spray, lasers, spin coating, carbon dioxide reduction, hydrothermal self-assembly, and nanotube slicing. Chemical vapor deposition can also be used according to different methods such as cold-wall, epitaxy, metal substrates (including ruthenium, iridium, nickel and copper), sodium ethoxide pyrolysis, and roll-to-roll manufacturing. Exfoliation, the main method of graphene production, can also be performed in a variety of ways including, but not limited to, electrochemical synthesis, molten salts, sonicating graphite at the interface of two immiscible liquids, most notably heptane and water, and sonication of graphite can also be performed in a simple liquid medium.

In one embodiment, a carbon-based material or nanomaterial such as graphene may be incorporated into an occlusive device through dispersion into a solution comprising a polymer or polymer composition and a solvent. The polymer or polymer composition may include, but is not limited to styrene maleic anhydride, styrene maleic acid, ethylene vinyl alcohol (EVOH), ethylene vinyl acetate, polyethylene oxide, polyethylene glycol (PEG), PLLA, PLGA, PLA, PGA, PVA, PDMS, PIPA, PEVA, PILA, PEG styrene, nylon, Teflon RFE, PEKEKK, FLPE, neoprene, PETE, Teflon FEP, Teflon PFA, PMP, methyl palmitate, NIPA, polycarbonate, polyethersulfone, polycaprolactone, polymethyl methacrylate, polypropylene, polyurethane, polystyrene, polyisobutylene, nitrocellulose, medical grade silicon, cellulose acetate butyrate, polyacrylonitrile, PLCL, chitosan, alginate, and mixtures thereof. Other polymers that are generally useful for forming an occlusion or embolism inside a vessel are known in the art and may be used in conjunction with the carbon-based material or nanomaterial. Non-limiting examples of polymers that may be used include those described in International Patent Application Publication No. WO 2015058169. Additional non-limiting examples include hydrogels such as polymethyl methacrylate, polyacrylonitrile, poly (carbonate-urethane), poly (vinylacetate), nitrocellulose, cellulose acetate, urethane, urethane/carbonate, polylactic acid, polyacrylamide (PAAM), poly (N-isopropylacrylamine) (PNIPAM), poly (vinylmethylether), poly (ethylene oxide), poly (ethyl (hydroxyethyl) cellulose), poly(2-ethyl oxazoline), poly(e-caprolactone), polydiaoxanone, polyanhydride, trimethylene carbonate, poly(β-hydroxybutyrate), poly(g-ethyl glutamate), poly(DTH-iminocarbonate), poly(bisphenol A iminocarbonate), poly(orthoester) (POE), polycyanoacrylate (PCA), polyphosphazene, polyethyleneoxide (PEO), polyethylene glycol (PEG), polyacrylacid (PAA), polyacrylonitrile (PAN), polyvinylpyrrolidone (PVP), polyglycolic lactic acid (PGLA), poly(2-hydroxypropyl methacrylamide) (pHPMAm), poly(vinyl alcohol) (PVOH), PEG diacrylate (PEGDA), poly(hydroxyethyl methacrylate) (pHEMA), N-isopropylacrylamide (NIPA), poly(vinyl alcohol) poly(acrylic acid) (PVOH-PAA), or natural polymers such as decellularized tissues, collagen, silk, fibrin, gelatin, hyaluronan, hyaluronic acid, cellulose, chitin, chitosan, dextran, casein, albumin, ovalbumin, heparin sulfate, starch, agar, heparin, fibronectin, fibrin, keratin, pectin, or elastin. Additional examples include those described in U.S. Patent No. 6,878,384, which discloses that hydrogels can be prepared by forming a liquid reaction mixture that contains a) monomer(s) and/or polymer(s) at least portion(s) of which are sensitive to environmental changes (e.g., changes in pH or temperature), b) a crosslinker and c) a polymerization initiator. Additional examples of hydrogels include those described in U.S. Patent Application Publication No. 20090053276A1 and U.S. Patent Nos. 6,703,047; 5,612,052; 5,714,159; 6,413,539; 4,804,691; 6,723,781; 5,866,554; 6,037,331; 6,514,534; 6,297,337; 6,514,535; and 5,702,717. In embodiments, the polymers can be prepared as a solution prior to administration into a body lumen. Additional polymers and hydrogels that can be used include those disclosed in International Patent Application Publication No. WO 2017083753, as well as in U.S. Patent Application Publication Nos. 20170136143 and 20170136144.

Higher molecular weight compounds (corresponding to longer polymer chains) allow for increased polymer flexibility. This is due to the fact that larger molecular weight chains allow for rotation of the chain around the single carbon-carbon bond.

In embodiments, the polymers can have a weight average molecular weight (M_{w}) or number-average molecular weight (Mₙ) ranging from about 1,000 to 1,000,000 as measured by GPC (gel permeation chromatography) with polystyrene equivalents, mass spectrometry, or other appropriate methods. In embodiments, the number-average molecular weight (Mₙ) or the weight average molecular weight (M_{w}) of polymers of the invention can range from about 1,000 to about 1,000,000 Daltons, such as from about 3,000 to about 60,000 Daltons, or from about 20,000 to about 90,000 Daltons, or from about 150,000 to about 900,000 Daltons, or from about 200,000 to about 750,000 Daltons, or from about 250,000 to about 400,000 Daltons, or from about 300,000 to about 800,000 Daltons, and so on. Further, the degree of polymerization of the polymers in embodiments can range from 1 to 10,000, such as from 50 to 500, or from 500 to 5,000, or from 1,000 to 3,000.

The chain length or degree of polymerization (DP) can have an effect on the properties of the polymers. In the context of this specification, the degree of polymerization is the number of repeating units in the polymer molecule. Included are polymers comprising from 2 to about 10,000 repeating units. Preferred are polymers comprising from 5 to 10,000 repeating units, such as from 10 to 8,000, or from 15 to 7,000, or from 20 to 6,000, or from 25 to 4,000, or from 30 to 3,000, or from 50 to 1,000, or from 75 to 500, or from 80 to 650, or from 95 to 1,200, or from 250 to 2,000, or from 350 to 2,700, or from 400 to 2,200, or from 90 to 300, or from 100 to 200, or from 40 to 450, or from 35 to 750, or from 60 to 1,500, or from 70 to 2,500, or from 110 to 3,500, or from 150 to 2,700, or from 2,800 to 5,000, and so on.

Further, the polymer may be cross-linked such as, for example, with a synthetic peptide, disulfide bonds, or the carbon-based nanomaterial itself. Carbon-based crosslinking can occur through ligands or binding partners that directly bind to the carbon allotrope. Additionally, different chemical functional groups can be introduced onto and into the carbon allotropes, such as but not limited to carboxylic acids, amines, alcohols, thiols, etc. Additional, chemistry then can be performed on these functional groups such that binding or crosslinking can occur by covalent reactions and non-covalent interactions such as but not limited to van der waals, pi-pi interactions, hydrogen bonding, dipole-dipole, chain entanglement, and mechanical trapping. Further, the polymer may be soluble in an organic solvent.

In embodiments, the carbon-based material or nanomaterial is suspended in a polymer dissolved in a solvent which includes, but is not limited to, saline or any other buffered aqueous solution, dimethyl sulfoxide, ethyl acetate, methanol, acetone, acetonitrile, tetrahydrofuran (THF), dioxane, and/or dimethylformamide (DMF), heptane, hexane, pentane, petroleum ether, benzene, toluene, o-xylene, m-xylene, p-xylene, ethanol, methanol, t-butyl alcohol, diethylene glycol, glycerol, ethylene glycol, chloroform, dichloromethane, 1,2-dichloroethane, hexafluoroisopropanol, deuterated solvents, or fluorocarbon solvents. According to other embodiments, the carbon-based material or nanomaterial is suspended into polymer that is not dissolved in solvent, and as such, it is a bulk preparation.

According to embodiments, the carbon-based material or nanomaterial is functionalized to make it biocompatible or otherwise more safe and effective, including the following carbon allotropes, but not limited to, functionalizing graphene or other carbon-based material or nanomaterial with the following ingredients. In embodiments, the carbon-based material or nanomaterial is functionalized with carboxylic acid (COOH) or carboxylic group, amine (NH2), ammonia (NH3) or ammonium, pristine, argon (Ar), silicon (Si), a fluorocarbon, nitrogen (N2), fluorine (F), oxygen, alkyl, cycloalkyl, aryl, alkylaryl, amide, ester, ether, sulfonamide, carboxylate, sulfonate, phosphonate, fluorocarbons, carbonates, nitro, halogens (bromine, chlorine, fluorine), boron, boronic acids, biomacromolecules including sugars and proteins, polymers such as polyethylene glycol (PEG) or pi-conjugated polymers, and supramolecular/coordination complexes including metal coordination complexes, and supramolecular complexes (e.g. π-π interactions with aromatics and pi-conjugated materials). These supramolecular complexes include non-covalent interactions such as host-guest chemistry/binding, hydrogen-bonding, van der Waals, and pi-pi stacking with small molecules, oligomers, polymers, polysaccharides, sugars, oligosaccharides, proteins, peptides, oligonucleotides, biomolecules, RNA/DNA, aptamers, biomolecules, derivatives of biomolecules, and other derivatives.

According to embodiments, the carbon-based material or nanomaterial is functionalized with peptides or antibodies. In one embodiment, the carbon-based nanomaterial is functionalized with S 19, also known as MHS-8, which is generated against sperm agglutination antigen-1 (SAGA-1). In other embodiments, the carbon-based nanomaterial is functionalized with antibodies to the 22 kD sperm protein, SP-22, which correlates with fertility and predicts fertility in males. *See* U.S. Patent No. 6,197,940. Antibodies to this protein are described in U.S. Patent No. 7,754,212. Functionalization with these antibodies may enhance the spermicidal properties of the carbon-based nanomaterial, and/or decrease the motility or fertility of sperm. Further, the carbon-based nanomaterial may be functionalized with antibodies that target ova, which antibodies include against SAS1B, also known as ovastacin. The antibodies may be monoclonal or polyclonal or subsequent fragments of these antibodies. Additionally, aptamers, DNA, RNA, PNA, peptides, proteins, enzymes, sugars, polysaccharides, small molecules, large molecules/polymers that provide targeting. In one embodiment, the functionalization allows for targeting within the reproductive space.

In one embodiment, the device is prepared by mixing a carbon-based nanomaterial such as a graphene-based compound into a polymer-solvent solution. For example, the polymer may be dissolved in an organic solvent, such as DMSO, at a concentration of 1 - 50 wt%, such as from 1 - 2 wt%, 2 - 3 wt%, 3 - 4 wt%, 4 - 5 wt%, 5 - 6 wt%, 6 - 7 wt%, and so on and so forth to 50 wt%. For ethylene vinyl alcohol, it is preferred to be dissolved at a concentration of 6 to 18% wt%, such as from 6 - 7 wt%, 7 - 8 wt%, 8 - 9 wt%, 9 - 10 wt%, 10 - 11 wt%, 11 - 12 wt%, and so on and so forth. For styrene maleic anhydride/acid, it is preferred to be dissolved at a concentration of 15-30 wt%, such as from 15 - 16 wt%, 16-17 wt%, 17 -18 wt%, 18 - 19 wt%, 19-20 wt%, 20 - 21 wt%, and so on and so forth to 30 wt%. The polymer may be dissolved in DMSO for at least 5 hours at 50 degrees Celsius. The carbon-based material or nanomaterial may be added at concentration of .01 wt% to 5 wt%, such as from .01 - .02 wt%, .02 - .03 wt%, .03 - .04 wt%, .04 - .05 wt%, .05 - .06 wt%, .06 - .07 wt%, and so on and so forth to 5 wt%, however, it is preferred that the carbon-based material or nanomaterial is added at a concentration of .01 - .1 wt%. In embodiments, it is preferred to have the carbon-based material, carbon-based nanomaterial, or carbon-based allotrope present in the end composition in an amount ranging from 10 ng/ml to 100 mg/ml, such as from 50 ng/ml to 50 mg/ml, or from 100 ng/ml to 1 mg/ml, or from 250 ng/ml to 75 mg/ml, or from 500 ng/ml to 30 mg/ml, or from 1 µg/ml to 10 mg/ml, or from 100 µg/ml to 800 µg/ml, or from 400 ng/ml to 500 µg/ml, or from 250 µg/ml to 900 µg/ml, and so forth, including any intermediate range or endpoint. Once the carbon-based material or nanomaterial is added, it may be dispersed homogeneously by vortexing, probe sonication, or ultrasonication bath. For example, with respect to ultrasonication, such a procedure may be performed from 10 degrees C to 70 degrees C, preferably between 20 and 25 degrees C, for 1-10 hours, preferably from 2-3 hours at a frequency set between 1-14 MHz preferably between 5-7 MHz. Ultrasonication is the preferred method for graphene dispersal due to its ability to uniformly disperse the graphene-based compound or graphene into the polymer-organic solvent solution. Uniform dispersion of the graphene-based compound or graphene is important for preventing agglomeration and keeping cytotoxicity at a minimum.

In one embodiment, the composition includes binding partners and/or ligand partners which facilitate cross-linking of the carbon-based material or nanomaterial with the polymer. In embodiments, the binding partners and/or ligand partners which facilitate cross-linking are aromatic compounds such as any substituted or unsubstituted C₄-C₁₀ aromatic compound, optionally with one or more carbon replaced by oxygen, nitrogen or sulfur, including for example naphthalene diimide terminated polymer X-linker (telechelic or star polymer.)

In one embodiment, the carbon-based materials or nanomaterials of the device include quantum dots. The quantum dots exhibit optical properties because they appear on the photoluminescent spectra and the UV Spectra. The quantum dots can be excited, furthering their effectiveness in bioimaging. In embodiments, the quantum dots allow the device of the invention to be imaged via modalities other than ultrasound, such as through applying a laser at an appropriate wavelength.

In one embodiment, the carbon-based material or nanomaterial of the device has altered tensibility and is thermosensitive and/or light sensitive. The device may be reversed by exposing it to near infrared light, visible light, or ultraviolet light. The light can cause the device to exhibit rapid change, such as shrinkage, and the device can expand when the light is turned off. In additional embodiments, the carbon-based material, such as graphene or quantum dots, facilitates the photoreversibility of the device by absorbing energy from light.

In embodiments, other constituents are included in the device. These may include additional contrast agents, imaging agents, therapeutic drugs, antimicrobials, anti-inflammatories, spermicidal agents, vasodilators, steroids, hormones, ionic solutions, proteins, nucleic acids, antibodies, or fragments thereof. The other constituents can provide additional contraceptive activity to the device. For example, the other constituents may produce an effect on sperm motility, viability, or fertility and may be a small molecule, protein, peptide, antibody, nucleic acid, or fragment thereof. Additionally, other constituents of the device such as the polymer and/or solvent can be innately spermicidal, such that no exogenous spermicidal agent is included.

In one embodiment, the device may be modified or cross-linked with fusion proteins, amino acid sequences, or peptides (natural or synthetic). In one aspect, the polymer component of the device may be modified with polyethylene glycol (PEG), where PEGylation may enhance the biocompatibility of the polymer. The polymer may be modified with an amino acid sequence. In another aspect, the amino acid sequence contains lysine residues which are cross-linked to the maleic acid or maleic anhydride groups. The amino acid sequence may be cleaved with an endo- or exo-protease. In one aspect, the amino acid sequence is a dipeptide. The addition of a protease causes the gel to de-precipitate, liquefy, or dissolve for reversal. In one aspect, the protease is found naturally in the human body. In one aspect, the protease is not found in the human body. The amino acid sequence and protease may be chosen from a database. In one aspect, the protease is papain, bromelain, actinidin, ficin, or zingibain. In another aspect, the di-amino acid scission site may only be cleaved by a bacterial protease. Preferably, the protease is injected in a solution form into the vas deferens to reverse, de-precipitate, liquefy, dissolve, or flush out the polymer gel.

In one embodiment, the device is a mesh-like structure that is formed by electrospinning a polymer-graphene (or other carbon-based material or nanomaterial) mixture. The mesh may be made using a mixture of a polymer such as styrene maleic anhydride or polycaprolactone (PCL). If PCL is used, it can be dissolved in a mixture of chloroform and DMSO, where the ratio of chloroform to DMSO is 90:10, 80:20, 70:30, 60:40, 50:50, 40:60, 30:70, 20:80, 10:90, 0:100; the preferred ratio of chloroform to DMSO is 50:50. The mesh may be made using a mixture of polymer and graphene-based compound or graphene. The polymer and graphene-based compound or graphene mixture may be ultrasonicated before electrospinning. The mesh can be formed using a voltage between 10- 20kV, ideally 15kV, a working distance of 10-25 cm, ideally 20 cm, and a flow rate of 0.5 ml/h-2 ml/h, ideally 1 ml/h. In an example of such a procedure, 1 ml of polymer solution is added to a syringe fitted with a blunt tip. Aluminum foil may be used as the collector site for the mesh. The collected fibers are placed in a vacuum desiccator for a period of 1-4 days, ideally 2 days. The mesh may then be implanted in a vessel, duct, interstitial space, or organ, such as by using a needle or a catheter. The mesh may then expand or constricts upon extrusion into the lumen; for example, the mesh may expand and bind or otherwise react with the epithelial cells of the vas deferens.

In embodiments, the electrospun device is a mixture of a polyester or polysaccharide, which allows for the device to expand upon contact with water. The mesh may be surrounded by or integrated with a hydrogel or part of a hydrogel composition/formulation. The hydrogel and/or mesh may be biodegradable or otherwise reversible. Further, the mesh may be coated in, is made of, or otherwise includes a pharmaceutical or composition with pharmacokinetic properties such as sustained release. Once implanted, the hydrogel may degrade over time or due to interaction with a solution (naturally occurring in the body or otherwise), leaving only the mesh in place.

According to embodiments, the mesh is inserted with a balloon catheter similar to a stent. The pore size of the mesh may be less than 3 microns or otherwise sized to prevent the flow of sperm or other cells. When inserted into the vas deferens, the mesh may render sperm infertile, immotile, or otherwise inviable upon interaction. When inserted into an artery leading to a tumor, the mesh may cause necrosis of said tumor. The mesh can be cylindrical, spherical, or any other shape.

According to other embodiments, the device is a fiber mat comprising graphene and/or polymeric material. In one aspect, the electrospun mesh is injected as small beads.

In one embodiment, the graphene in the device is composed of 1-10 sheets. In one aspect, pure graphene is one layer thick. In one embodiment, the graphene nanoplatelets range from 1-10 layers. In one aspect, the nanoplatelets are from 1-10 µm in diameter.

The physical and chemical properties of the device may be affected by the inclusion of carbon-based materials or nanomaterials, such as graphene-based compounds or graphene. For example, the viscosity and/or rheometry of the solution may be altered (e.g., the viscosity and/or rheometry may increase). Further, the thermal conductivity, stability, electrical conductivity, barrier properties, stiffness, young's modulus, elasticity, durability, tensile strength, friction, and toughness may change; for example, they may increase. Still further, the gel's brittleness, fatigue, creep, degradation, and porosity may change; for example, they may decrease.

In one embodiment, a carbon-based material or nanomaterial such as a graphene-based compound or graphene within the gel or mesh reinforces the gel's or mesh's ability to occlude cells including sperm and/or oocytes. For example, the matrix size of the gel or mesh may be decreased due to the graphene making it difficult for cells including sperm and/or oocytes to traverse through. The pores on the surface of the gel or mesh may be uniform or the porosity may be changed, either decreased or increased, with the addition of a graphene-based compound or graphene or other carbon-based nanomaterial. In one embodiment, the pores are less than about 3 microns in size (thus, preventing sperm from traveling through). Further, some or all fluid, some or all small particles, and some or all macromolecules, may pass through the pores (other than what is meant to be occluded).

In one embodiment, the device may be used as a drug delivery agent to provide for sustained release of a drug from the device; the drug may be included around, inside, included within, or outside the device. In one embodiment, the drug may be, but is not limited to, therapeutic drugs, antimicrobials, anti-inflammatories, steroids, drugs, hormones, ionic solutions, proteins, or nucleic acids, or fragments thereof. The device may also be used for delivery of genetic material such that DNA, RNA, siRNA, aptamer, or other genetic material is released from the device through the pores or otherwise. Other applications for the device include control of cell growth, or as a scaffold for cells *in vivo* or *in vitro*, or otherwise for tissue engineering. The device may increase cell adhesion and proliferation. The different binding interactions and their influence on stem cell growth and differentiation are attributed to different degrees of Π-Π stacking and electrostatic and hydrogen bonding mediated by graphene and graphene oxide (*see* Lee, W. C., Lim, C. H. Y., Shi, H., Tang, L.A., Wang, Y., Lim, C. T., & Loh, K. P. (2011). Origin of enhanced stem cell growth and differentiation on graphene and graphene oxide. ACS nano, 5(9), 7334-7341). For example, in one embodiment, the device allows for accelerated stem cell differentiation due to the strong noncovalent binding abilities of the carbon-based nanomaterial. This allows it to act as a preconcentration platform for stem cell inducers, which in turn accelerates the stem cells growing on it toward the appropriate lineage. The carbon-based nanomaterial portion of the device may provide antibacterial, antiviral, and/or antimicrobial properties for the gel, mesh, or device.

In one embodiment, the gel, mesh, or device taught herein may contain an ultrasound-contrast agent such as microbubbles to render the gel, mesh, or device echogenic or visible under ultrasound. The microbubbles may contain a carbon-based nanomaterial such as a graphene-based compound or graphene in the shell or what the shell is enclosing. In one embodiment, the carbon-based material or nanomaterial are innately echogenic. The microbubbles may be prepared via double emulsion method or using a microfluidic chip. Further, the microbubbles may have different-longer or shorter-stability because of the inclusion of the carbon-based material or nanomaterial. The shell may be a mixture of a polymer and/or lipid(s) with the carbon-based material or nanomaterial. The microbubbles with the carbon-based nanomaterial may be used for gene or drug delivery, such as through thermal targeting to constrict the polymer. Further, the microbubbles with the carbon-based nanomaterial can include a reversal agent that may de-polymerize or dissolve the gel, mesh, or device upon release.

The ultrasound contrast agent can be microbubbles, or any other known ultrasound contrast agent or which becomes known in the art. Ultrasound contrast agents have been reviewed in the literature (*see* Calliada F, et al., "Ultrasound contrast agents: basic principles", Eur J Radiol. 1998 27 Suppl 2:S157-60 and Cosgrove D, "Ultrasound contrast agents: An overview", Radiology 2006 Volume 60, Issue 3, Pages 324-330). In one embodiment, the microbubbles decrease the lateral and axial resolution (as calculated by the full-width, half-maximum formula), thereby enhancing the visibility of the gel on the ultrasound. The addition of microbubbles allow for the gel to be echogenic (visible on ultrasound) for extended duration.

In embodiments, the ultrasound contrast-enhancing agent includes gas-containing microbubbles or microspheres. The microbubbles may also be hollow or porous. The gas may include a mixture or combination of gases (e.g. air), or any inert gas, such as nitrogen, argon, perfluorocarbon, and the like. The microbubbles may have a shell which includes as components a polymer, a lipid, a protein, a surfactant, a monosaccharide, a polysaccharide, or glass.

Useful polymers for microbubbles may include, but are not limited to, polystyrene, neoprene, polyetherether 10 ketone, polyethylene, polypropylene, polyetherketoneetherketoneketone (PEKEKK), nylon, TEFLON^{®} TFE, polyethylene terephthalate (PETE), TEFLON^{®} FEP, TEFLON^{®} PFA, and polymethylpentene (PMP). The polymers may be insoluble in DMSO. Useful polysaccharides for microbubbles include, but are not limited to, cellulose, cellophane, or carboxymethyl cellulose, or any derivative thereof. An example of a protein constituent of a microbubble shell includes albumin, and an example of a saccharide constituent of a microbubble shell includes galactose. Additionally, the microbubbles may include multiple constituents.

In embodiments, the microbubbles can be formulated to contain additional agents or drugs, including, but not limited to, therapeutic drugs, antimicrobials, anti-inflammatories, steroids, drugs, hormones, ionic solutions, proteins, peptides, antibodies, or nucleic acids, or fragments thereof. The additional drugs or agents can be controlled through sustained release. The molecules can be conjugated to the microbubbles of the invention or included as internal components of the microbubbles. The molecule may be a small molecule, protein, a peptide, antibody, or a ligand which targets sperm to render the sperm immotile, infertile, or inviable.

In embodiments, the microbubbles or microspheres of the invention can vary in size, or can be provided in a fairly uniform size range. The microbubbles can range in size from about 0.10 to about 1,000 µm in diameter. However, a substantially uniform size range is preferred to provide maximum contrast. For example, the microbubbles can be provided in a size ranging from about 1-2, 2-3, 3-4, 4-5, 5-6, 6-8, or 8-10 µm in diameter. Additionally, the microbubble shell thickness can vary in size. In embodiments, the microbubbles are provided at a concentration ranging from about 1 x 10² to about 1 x 10⁹ microbubbles/ml, including 1 x 10³, 1 x 10⁴, 1 x 10⁵, 1 x 10⁶, 1 x 10⁷, and 1 x 10⁸ microbubbles/ml. In some embodiments, the microbubbles are innately present in the composition. In other embodiments, the microbubbles are fabricated separately and added to the composition.

For example, microbubbles or microspheres can be crosslinked to the polymer of the composition. The microbubbles or microspheres can be added separately to the gel solution and mixed to form a homogenous solution. The solution can be mixed by stirring. For example, the microbubbles can be formed by mixing a polymer-DMSO solution to form a foam solution. In other embodiments, a double emulsion method can be used (*see* El-Sherif, D. M., & Wheatley, M. A. "Development of a novel method for synthesis of a polymeric ultrasound contrast agent", Journal of Biomedical Materials Research Part A, (2003) 66A(2), 347-355), as well as by pumping the polymer-DMSO mixture through 2 syringes and 3-way stopcock, a method which has been employed for producing agitated microbubbles, but only in saline solutions (*see* Attaran, R. R, "Protocol for Optimal Detection and Exclusion of a Patent Foramen Ovale Using Transthoracic Echocardiography with Agitated Saline Microbubbles" (2006), Echocardiography (Mount Kisco, N.Y.), 23(7), 616-22). Using the double emulsion method according to the present invention, air-filled polystyrene and polyvinyl alcohol (PVA) microbubbles were produced. The mixing can also be accomplished by transferring the solution between syringes (from one to another). Further, specific size ranges of microbubbles can be isolated by differential centrifugation and added to samples of polymer solutions, and the samples may be subject to ultrasound imaging. In this way, the maximum echogenicity (contrast) of the microbubbles in different size ranges can be determined.

In one embodiment, microbubbles are prepared by pumping the polymer-DMSO solution through 2 syringes connected by a 3-way stopcock with swivel male luer lock. The number of pumps can be from 1-200, such as from 1-2, 2-3, 3-4, 4-5, 5-6, and so on. The lateral and axial resolution of the gel decreases with number of pumps, indicating that more microbubbles are formed with increased number of pumps. The volume of air in the syringe loaded prior to the pumps can be varied from 0 - 3 mL. In one example, performing 80 pumps with a loaded air-volume of .75 mL yields a low lateral and axial resolution (high visibility of the gel). This combination also yields the smallest decrease in visibility over time. In some embodiments, the microbubbles that are formed have no shell and comprise air. Larger microbubbles are found at the top of the polymer solution in the syringe. In another aspect, the smaller microbubbles are found at the bottom of the polymer solution in the syringe. In embodiments, the polymer-DMSO-microbubble solution is injected into a bodily duct (*e.g*., vas deferens).

In one embodiment, the microbubbles are prepared through the following double emulsion procedure. A polymer of interest is dissolved in organic solvent such as chloroform. Water is added and the solution is sonicated. Further, a surfactant such as PEG stearate is added to separate the water droplets within the bulk of organic solvent and polymer. The emulsion is poured into a polyvinyl alcohol (PVA) solution and homogenized. Preferably, the PVA solution is 5%, but can range from about 1% to about 80%, such as from 1 % to 2%, 2% to 3%, 3% to 4%, 4% to 5%, 5% to 6%, 6% to 7%, and so on. The homogenized solution is poured into isopropanol. Preferably, the isopropanol solution is 2%, but can range from around 1% to about 50%, such as from 1% to 2%, 2% to 3%, 3% to 4%, 4% to 5%, 5% to 6%, 6% to 7%, and so on. The mixture is stirred for one hour at room temperature with stirring times that may vary. The mixture is then centrifuged to obtain a pellet of microbubbles. The pellet is resuspended in water and re-centrifuged. The solution is then lyophilized to remove the water from inside the microbubbles.

In one embodiment, perfluorocarbon gas (including perfluoromethane, perfluoroethane, perfluoropropane, perfluorobutane, perfluoropentane, and/or other such perhalocarbon gases) is contained within the microbubbles, preferably as a contrast agent. In one aspect, double emulsion is used to prepare microbubbles containing perfluorocarbon gas. The gas may be sonicated within a polymer and organic solvent solution. In one example, the emulsion is poured into around 5% PVA, but can range from around 1% to around 50%, such as from 1% to 2%, 2% to 3%, and so on. In another example, the emulsion is poured into 2% isopropanol, but can range from around 1% isopropanol to around 35%, such as from 1% to 2%, 2% to 3%, and so on. The solution may be allowed to stir at room temperature for around one hour. After around one hour, the mixture is centrifuged, re-suspended in water, and centrifuged again to obtain a pellet of microbubbles. *See* U.S. Patent No. 5,695,740.

In one embodiment, the microbubbles include polyvinyl alcohol (PVA). The PVA bubbles can be cross-linked. The method by which the bubbles are prepared can include dissolving PVA in a solvent. In one example, an oxidizing agent is added, followed by double emulsion. The ends of the PVA polymeric chains are functionalized with aldehyde groups before continuing double emulsion. *See* Chinese Patent No. 103724638 A and U.S. Patent Application Publication No. US 20100158813 A1.

In one embodiment, the shells of the microbubbles are made of polystyrene. In one aspect, the molecular weight of the polystyrene ranges from 35,000 - 400,000 daltons, such as from 35,000 to 40,000 daltons, from 40,000 to 45,000 daltons, from 45,000 to 50,000 daltons, from 50,000 to 55,000 daltons, and so on.

In one embodiment, the microbubbles include one or more spermicides, such as those discussed herein. In one aspect, the microbubbles contain a degradation agent such as a reducing agent (e.g., glutathione) to assist in reversal of the polymer gel.

In one embodiment, the carbon-based nanomaterial itself increases the echogenic properties of the device such that it can be visualized by way of an imaging modality, such as ultrasound imaging. In these embodiments, additional contrast agents such has microbubbles may be added, or may not be required. In embodiments, the echogenicity of the carbon-based nanomaterial within the compositions may be tested *in vitro* under ultrasound such as in 96-well plates.

According to embodiments, the device is formulated to have a specific porosity once it polymerizes *in situ* to form a gel. For example, the porosity can be tailored to allow passage of fluids (as well as constituents such as proteins, nutrients, etc.) in a lumen such as the vas deferens while blocking sperm cells. In embodiments, the pore diameter is less than about 3 µm (*e.g.* the approximate width of the head of a human sperm cell). In embodiments, the pore diameter of the formed polymer can range from 0.001 nm to 3 µm, such as from 0.001 nm to 1 µm. In other embodiments, the pore diameter can range from 0.01 nm to 100 nm. In other embodiments, the pore diameter can range from about 1 nm to about 1 µm. In other embodiments, the pore diameter can be 0.01, 0.02, 0.03, 0.04, 0.05, 0.06, 0.07, 0.08, 0.09, 0.10, 0.15, 0.20, 0.25, 0.30, 0.35, 0.40, 0.45, 0.50, 0.55, 0.60, 0.65, 0.70, 0.75, 0.80, 0.85, 0.90, 0.95, 1, 2, 3, 4, 5, 6, 7, 8, 9, 10, 15, 20, 25, 30, 35, 40, 45, 50, 55, 60, 65, 70, 75, 80, 95, 90, 95, or 100 nm. In other embodiments, the pore diameter is at least the size of an atom (0.5 nm). Specific pore sizes can be targeted to provide an optimum porosity that provides maximum flow of fluid while blocking the flow of sperm cells.

Another embodiment of the invention provides a method of occluding a lumen comprising: identifying a lumen; administering ultrasonic energy to image the lumen, and performing one or more or all of the following steps optionally under guidance of ultrasound imaging: measuring one or more dimensions of the lumen; percutaneously placing a needle or catheter or portion thereof into the lumen; administering a composition of the invention into the lumen through the needle or catheter; confirming formation of an occlusion inside the lumen; and/or determining one or more dimensions of the occlusion inside the lumen.

Another embodiment of the invention provides a method of occluding a body lumen comprising: imaging a body lumen using ultrasound; and percutaneously placing a needle or catheter or portion thereof into the lumen; administering a composition of the invention into the lumen through the needle or catheter; and allowing the composition to form an occlusion in the lumen.

Another embodiment of the invention provides a method of occluding a body lumen comprising: imaging an animal or human body lumen to view an image of the body lumen; administering a composition of the invention into the body lumen; polymerizing the substance or forming a mass from the substance in the body lumen; and imaging the substance or mass.

The body lumen may be an artery, a vein, a vas deferens, a fallopian tube, a uterus, a duct, such as a bile duct, hepatic duct, or pancreatic duct, interstitial space, or an organ.

In embodiments, the device is administered as a solution into the body lumen at a rate or amount which dictates the shape and length of the occlusive plug that forms. The rate of administration can be constant or variable. For example, the solution can be injected or infused at a rate of 0.001 cc/min (1 µl/min) to 1.0 cc/min (1 ml/min), including 0.001, 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.15, 0.2, 0.25, 0.30, 0.35, 0.40, 0.45, 0.50, 0.55, 0.60, 0.65, 0.70, 0.75, 0.80, 0.85, 0.90, 0.95, and 1.0 cc/min. It is preferable that the physician injects the polymer material at a constant rate. With a relatively slow injection rate (e.g. about 0.1 to 0.2 cc/min), the polymer will form a tightly-packed, cylindrical gel. At a relatively fast injection speed (e.g. greater than about 0.50 cc/min), the polymer gel can be more string-like and may not fully occlude the lumen. A pressure-controlled syringe or device may be used to ensure a constant injection speed.

Total volumes administered can vary from about 1 µl to about 5000 µl (5 ml), including 1, 2, 3, 4, 5, 6, 7, 8, 9, 10, 15, 20, 25, 30, 35 ,40, 45, 50, 55, 60 ,65, 70, 75, 80, 85, 90, 95, 100, 110, 120, 130, 140, 150, 160, 170, 180, 190, 200, 225, 250, 275, 300, 350, 400, 450, 500, 550, 600, 650, 700, 750, 800, 850, 900, 950, 1000 uL, and so on.

The rate of administration and total volume administered can depend on the size of the body lumen in terms of diameter and length, as well as the composition and properties of the polymer solution in terms of molecular weight and concentration of the polymer, viscosity, gelation temperature, rate of polymerization, and desired length of the occlusion. Such is within the capabilities of a skilled artisan. In one aspect, the volume and rate injection is low enough that the polymer does not leak into the vas wall or rupture the vas.

In embodiments, the length of occlusion produced in the body lumen as a result of administering the occlusive substance can range from 0.1 - 5 centimeters in length, including 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, and 5.0 cm in length. In embodiments, the length of the occlusion formed in the lumen is longer than the smooth muscle fibers of the vessel wall so that the plug is not dislodged by peristaltic contraction. Preferably, the polymer gel plug formed in the lumen is rigid enough so that the peristaltic contraction of the vessel (such as the vas deferens) does not break, dislodge, or otherwise affect the safety and/or efficacy of the polymer gel. According to one embodiment, the injection volume and length of polymer plug that forms is determined such that the subject becomes severely oligospermic or azoospermic as determined by analysis of semen samples. Upon administration, polymerization of the occlusive polymer can be monitored in real time using ultrasound.

In embodiments, ultrasound is used to image a vessel such as the vas-deferens and the occlusive device during and after placement inside the vessel. Ultrasound based imaging is a painless and convenient diagnostic method that functions by projecting sound waves into the body, and then measuring the refraction, reflection, and absorption properties of the imaged-tissue to assess fine structure. Essentially, the way in which certain structures reflect sound waves allows for the generation of an image of the underlying organs and tissues. For instance, ultrasound imaging works best on mechanically more elastic, sound conducting tissues. Calcifications in the body (such as bone, plaques, and hardened tissues) provide degrees of acoustic impedance that makes it difficult to image structures lying below them.

Ultrasound is an ideal candidate for imaging the tissues in the male reproductive system. First, ultrasound imaging is non-invasive and safe. There is no associated ionizing radiation produced with ultrasound as found in X-Ray, PET, and X-Ray imaging. Second, the male reproductive system, specifically the scrotum, does not contain bone, plaques, or hardened tissues which limit acoustic impedance. Finally, preparing a patient for ultrasound imaging is as simple as shaving the area of interest, cleaning the area of interest, applying an ultrasound-conducting fluid interface gel to the surface of the skin, and applying the ultrasound probe in the correct orientation and position. Therefore, ultrasounds are commonly found in urology clinics and are used primarily for imaging the scrotum and penis.

Various frequencies can be used for imaging the vas deferens and/or device, including contrast-pulse sequencing mode (7 MHZ), B-Mode imaging (14 MHZ), and frequencies in between. Other possible ultrasound modes that can be used for the inventive methods include 2D mode, fusion, harmonic imaging (THI), color mode or color power angio, CW doppler mode, PW doppler mode, M-Mode, anatomical M-mode (live or frozen image), B-Mode, color tissue doppler, PW tissue doppler, panoramic imaging, 3D/4D imaging, and dual imaging. In some embodiments, the frequencies are between 1 and 20 MHZ, including 1, 2, 3, 4, 5, 6, 7, 8, 9, 10, 11, 12, 13, 14, 15, 16, 17, 18, 19, or 20 MHZ. Additionally, the ultrasound can be delivered at different intensities, such as between 0.1 to 1 W/cm², including 0.1, 0.2, 0.3, 0.4, 0.5, 0.6, 0.7, 0.8, 0.9, and 1.0 W/cm². Additionally, the ultrasonic energy can be delivered at a specific power, such as 0 to 20 Watts of energy, including 0, 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20 Watts. Additionally, the ultrasonic energy can be delivered in pulsed or continuous mode. The ultrasound can be delivered through an ultrasound unit. The ultrasound unit can be portable. An example of a portable ultrasound unit for scrotal imaging is the LOGIQ V2, manufactured by GE Healthcare (Little Chalfont, United Kingdom). Another example of an ultrasound unit for scrotal imaging is the ClearVue 350 by Philips (Amsterdam, Netherlands).

According to embodiments, various ultrasound probes or transducers can be used for ultrasound imaging the vas deferens, including sector (phased array), linear and convex transducers. Ultrasound probes and their selection have been discussed in the literature (*see* T.L. Szabo et al., "Ultrasound Transducer Selection in Clinical Imaging Practice", Journal of Ultrasound in Medicine, 2013, 32(4):573-582). Ultrasound transducers differ according to their piezoelectric crystal arrangement, physical dimensions, shape, footprint (aperture), and operating frequency. It is within the ability of a skilled artisan (e.g. urologist or ultrasound technician) to choose a transducer with appropriate characteristics to image the area of the vas deferens that has been isolated. A hand-held probe may be chosen for imaging that is small enough to image the vas without interfering with other aspects of the procedure such as administration of the occlusive substance.

Transducers are multi-frequency, meaning the frequency can be switched electronically between a range of frequencies (e.g. abdominal transducers have 2-6 MHz). It is important for the user to select the highest frequency which adequate depth of penetration for the anatomic area of interest. In general, the higher the frequency of the transducer, the greater than axial resolution and better the anatomic representation of the image. However, there is a tradeoff between frequency and depth of penetration. For imaging the testis, because of the close proximity of the organ to the surface of the skin, imaging can be performed with high frequency transducers such as a linear array transducer of 12-18 MHz.

There are many factors that impact the image quality. Parameters and settings may be modified by the user of the ultrasound in order to adjust and manipulate the image including: gain, time-gain compensation, frequency, depth/size, field of view, and cine function. A "good quality image" includes: (1) sufficient and uniform brightness, (2) is sharp and in focus, (3) adequate size, and (4) is oriented and labeled for documentation purposes. Furthermore, selection of a transducer is critical for maximizing image quality. Linear array transducer probes produce a rectangular image whereas a curved array transducer produces a trapezoidal shape. Linear array transducers are most commonly used in urology for imaging the testes and male genitalia. However, a curved array transducer can be helpful in visualizing both testes simultaneously.

In regards to safety, the FDA advises that the mechanical index (MI) and thermal index (TI) are kept below 1.90 and 6 degrees C, respectively.

In embodiments, the present invention relates generally to methods of administration of a composition of the invention into any lumen in the body, such as the lumen of the vas deferens, optionally under guidance of ultrasound through non-surgical or surgical routes of administration. In a preferred embodiment, the substance is administered percutaneously. Further, the present invention contemplates any therapeutic or diagnostic aspect of the methods of the invention that can be appreciated by a skilled artisan.

For example, one embodiment provides a method which includes non-surgically or surgically isolating a vas deferens of a subject, placing an ultrasound probe on or near the vas deferens and administering ultrasonic energy to image a lumen portion of the vas deferens, and optionally under guidance of ultrasound imaging percutaneously placing a needle or catheter or portion thereof into the lumen portion of the vas deferens, and administering a composition of the invention into the lumen portion of the vas deferens through the needle or catheter.

The imaging agent can be detectable by way of any form of energy used in diagnostic imaging, such as, for example, by way of radiofrequency, photoacoustic, infrared, or ultrasonic energy. For example, the imaging agent can be or include an ultrasound contrast agent such as microbubbles or can be or include a dye, quantum dots, or other excitable substance. Additionally, the composition of the invention can be or include a therapeutic agent. In one particular embodiment, the agent is therapeutic in the sense that it produces a desirable contraceptive effect. The therapeutic agent can include any non-occlusive substance, such as small molecules, antibodies, peptides, proteins, nucleic acids, and the like.

In one particular embodiment, the composition of the invention is a polymer comprising a carbon-based nanomaterial, and the polymer is administered as a solution and results in polymerization *in situ* to form an occlusion in the vas deferens. Particularly advantageous embodiments of the present invention for which there is a critical need for include methods of vas-occlusive contraception that are minimally invasive, highly accurate, reproducible, safe, long-lasting, and easy to implement in an outpatient setting. According to embodiments, these advantages stem from the use of ultrasound imaging throughout at least a portion of the procedure.

The inventors' advantageous implementation of ultrasound to guide the placement of a device or composition of the invention into the lumen of the vas deferens has not been previously established in the contraceptive arts. Even further, the use of a non-surgical or surgical method for isolating the vas deferens and placement of a contraceptive in the lumen by way of percutaneous injection or controlled intra-vasal infusion is a significant improvement over previous vas-occlusive procedures in development, which generally require an incision to isolate the vas deferens. Moreover, the inventive procedure can be performed by a physician using local anesthesia in an outpatient setting very rapidly, in as little as five minutes, thus minimizing any discomfort to the subject. Moreover, the inventive procedure requires minimal training to perform, such that it can be performed by any medical practitioner familiar with ultrasound imaging. Even further, while the inventive procedure provides long-lasting contraception, it is easily reversible by way of a similar procedure. These advantages of the invention, only some of which are discussed herein, will be further apparent as various embodiments and features of the invention are described.

One embodiment of the invention includes a method of vas-occlusive contraception. The method can include non-surgically or surgically isolating a vas deferens of a subject in need of contraception, placing an ultrasound probe on or near the vas deferens and administering ultrasonic energy to image the lumen of the vas deferens, and performing at least one of the following steps optionally under guidance of ultrasound imaging, such as (a) determining one or more dimensions of the lumen of the vas deferens (b) percutaneously placing a needle or catheter or portion thereof into the lumen of the vas deferens, (c) administering a composition of the invention into the lumen of the vas deferens, (d) confirming formation of an occlusion inside the lumen as a result of administering the substance, and (e) determining one or more dimensions of the occlusion inside the lumen portion.

According to embodiments, the non-surgical or surgical isolation of the vas deferens includes use of a vas-fixation clamp to grip the vas deferens through the skin of the scrotum, and/or subcutaneous isolation through the physician's fingers. For example, the vas deferens can be isolated through the use of the "three-finger technique," in which the non-dominant hand is used to manipulate the vas into a subcutaneous position (*see* Stockton DM et al. "No-scalpel vasectomy: a technique for family physicians." Am Fam Physician. 1992;46:1153-67; Clenney TL, "Vasectomy Techniques," Am Fam Physician. 1999; 60(1):137-146). Alternatively or in addition, a vas-fixation clamp can be used to secure the vas deferens in a sub-cutaneous position. Further, the vas clamp can contain an additional component that guides the needle into the lumen of the vas deferens. Additionally, the needle may include a ball-and-socket joint which allows for easier manipulation of the needle.

According to embodiments, a composition of the invention can be administered into the vas deferens of both sides by way of percutaneous injection or controlled intra-vasal infusion into the vas, optionally under the guidance of ultrasound imaging. For example, the composition can be dissolved in a solvent, preloaded in a syringe, and injected into the lumen of the vas deferens by way of a needle or catheter. The needle or catheter is chosen to be of a size that fits inside the lumen of the vas deferens. For example, one publication has estimated the lumen of the vas deferens in humans to vary from 0.25 to 1.2 mm in diameter, while having an external diameter of between 2 and 4 mm (*see* E.S. Hafez, P. Kenemans, "Atlas of Human Reproduction: By Scanning Electron Microscopy," 1982, MTP Press, Hingham, MA). Thus, the inner diameter (lumen) of the vas is only a small portion of its outer diameter. However, various other studies have shown that the inner diameter can dilate as large as 1.8 mm (Liu, X. et al.). The outer portion of the vas is made up of layers of smooth muscle. This size differential underscores the need for ultrasound imaging to confirm successful administration of the vas-occlusive substance into the lumen rather than "off-target" administration into the smooth muscle. The size of the needle or catheter can be chosen based on the estimated size of the vas from the literature, or determined by imaging the dimensions of the vas lumen of the subject through ultrasound. In embodiments, the size of the needle can be between 18 gauge to 34 gauge, depending on the estimated or determined diameter of the vas lumen of the particular species that is the target of contraception. In other embodiments, the size of the needle is between 21 gauge and 31 gauge. In other embodiments, the size of the needle is at least 23 gauge, such as between 23 gauge and 29 gauge. In another aspect, the needle that is used to deliver the injection solution contains bores on the side, which allow for the solution to be excreted around the needle, in addition to the bevel.

This disclosure reports that ultrasound is the ideal imaging modality for performing a guided injection into the vas deferens. The relatively shallow depth at which the vas deferens sits allows for easy identification by a medium or high frequency ultrasound. Ultrasound is rarely used in clinical applications for imaging the vas deferens. Thus, prior to the present disclosure, methods for optimal imaging of the vas deferens were limited. To the best of the knowledge of the present inventors, ultrasound-guided, percutaneous injection into the vas deferens has never been performed. Optionally using ultrasound as guidance for performing percutaneous vas injections is critically needed because: 1) every subject has different morphometric measurements of the vas (*e.g.* outer and inner diameter, depth, length), 2) the physician or other professional (*e.g.* technician, veterinarian, etc.) performing the procedure can visualize that the needle is inside the vas lumen as opposed to the smooth muscle layers of the vas, 3) the physician can visualize the polymer solution being injected into the lumen, 4) the physician can visualize the polymer forming a hydrogel *in situ* in real time, 5) the physician can observe the length of the gel plug, confirming that enough of the material was injected, 6) the physician can perform routine "check-ups" on the composition using ultrasound days, weeks, or months after the composition is inserted, 7) the physician can locate the gel prior to reversal, 8) the physician can perform the reversal through ultrasound-guided, percutaneous injection, and 9) the physician knows that the reversal was successful if the gel is no longer visible on the ultrasound.

According to embodiments, the physician isolates the vas deferens using a finger technique and secures it to the scrotal skin using a vas clamp. In one embodiment, an ultrasound probe is placed on the vas deferens before, during, and/or after administration of the occlusive substance. The probe can be placed parallel to the vas such that the lumen will be visualized with a longitudinal view. In this view, the length of the vas can be determined as well as the inner and outer diameter. Alternatively or in addition, the probe can be placed perpendicular to the vas deferens such that the lumen of the vas will be visualized in axial/transverse view. In this mode, it is easy for the physician to discern the depth of the vas as well as determine the inner and outer diameter length.

According to one embodiment, the physician administers a vasodilator locally in the area of the secured vas deferens prior to administration of the occlusive substance. The vasodilator diffuses to the smooth muscle of the vas and causes it to relax, thereby expanding the lumen to a dilated state. This pharmacologic dilation of the vas can facilitate the procedure by 1) providing greater visibility of the lumen of the vas under ultrasound and 2) providing a larger target for insertion of a needle or catheter into the lumen, thereby reducing the chances of "off-target" insertion into the smooth muscle of the vas. Vasodilators are known in the art, including nitric oxide donors (*e.g.* nitroglycerin), acetylcholine, prostaglandins, papaverine, calcium channel blockers, phosphodiesterase type 5 (PDE-5) inhibitors, and the like. The vasodilator can be administered with an anesthetic agent or be present in the solution of the occlusive substance. In one embodiment, the vasodilator is administered percutaneously. In other embodiments, the vasodilator is administered topically. In other embodiments, no vasodilator is administered.

According to embodiments, a local anesthetic agent is administered in the area of the vas deferens prior to administration of the occlusive substance. Various local anesthetic agents are known, including amino esters such as procaine (Novocaine), tetracaine (Pontocaine), benzocaine, as well as amino amides such as lidocaine, mepivacaine, bupivacaine, and etidocaine. To avoid constriction of the vas deferens lumen, it is preferred that the local anesthetic agent be substantially devoid of vasoconstrictive activity, and that no vasoconstrictive agents such as epinephrine be administered with the anesthetic. The anesthetic can be administered topically or percutaneously.

Once the lumen is visualized, the physician percutaneously inserts a needle, cannula, catheter, or needle-catheter into the lumen under optional guidance of ultrasound imaging and administers (*e.g.* injects) a composition of the invention into the lumen. The injection process may be performed towards the direction of the testes, which would be against the flow of seminal fluid and assist in gel formation, or in the direction of the prostate. The region of the vas where the injection can be performed is either in the scrotal region or supra-scrotal region before the vas extends into the pre-pubic region. An echogenic needle can be used, which facilitates visual confirmation that the needle is inside the lumen via ultrasound before administration. Alternatively, an ultrasound with magnetic field needle guidance such as the eZono® 4000 (eZono AG, Jena, Germany) can also be used to guide the needle more precisely into the lumen in axial mode. The ultrasound may also be a handheld, portable ultrasound.

If the composition is echogenic, it will be seen being injected into the lumen. Then, the physician will be able to witness the composition form a hydrogel in the vas deferens in real-time.

After the gel "plug", or occlusion, forms, the physician can confirm the procedure was done properly by ultrasound imaging the gel. Axial mode can be used for viewing to determine if the gel completely occluded the vas, whereas longitudinal mode can be used determining the length of the vas-occlusive gel plug. In this way, the dimensions (*e.g.* length, width, and diameter) of the occlusion can be determined through ultrasound imaging. If the occlusion is not of sufficient size in terms of diameter and length, additional material can be administered inside the vas deferens lumen, until the physician confirms administration of an appropriate amount of vas-occlusive material through ultrasound imaging.

Thus, one particular embodiment of the invention provides a method of vas-occlusive contraception which includes:
Non-surgically or surgically isolating the vas deferens in the scrotum such as by using a three-finger type technique;
Optionally, administering anesthesia to the subject, such as by administering local anesthesia to the vasal nerve region (*e.g.* vasal block);
Raising the vas deferens and securing it, such as by securing the vas deferens to the scrotal skin using a vas-clamp, making the vas as superficial as possible;
Placing an ultrasound probe on the vas deferens and administering ultrasonic energy to the vas deferens to visualize the vas deferens lumen in longitudinal and/or axial view; and, by way of ultrasound imaging:
   Measuring one or more dimensions of the vas deferens lumen;
   Placing a needle into the vas deferens and confirming placement of the needle or catheter or a portion thereof into the lumen;
   Percutaneously administering a vas-occlusive polymer solution into the lumen;
   Confirming formation of a polymer occlusion inside the lumen; and
   Determining one or more dimensions of the occlusion in longitudinal and/or axial view.

The above procedure can then be repeated on the contralateral side.

For example, in one embodiment, the general area of the scrotum is shaved and an ultrasound-conducting fluid interface gel is applied to the scrotum. Then, an ultrasound probe is placed over the vas, ultrasonic energy is administered, and the physician percutaneously injects the vas-occlusive substance into the vas deferens optionally under the guidance of ultrasound imaging. Occlusion of the vas deferens by way of a vas occlusive plug after the procedure is performed occurs such that sperm cells are blocked from progressing through the lumen of the vas deferens.

It is predicted that the inventive methods will be significantly quicker than a typical vasectomy due to the fact that no incision or exteriorization of the vas is required. Furthermore, the inventive methods skip the step of removing the sheath that surrounds the vas, which occurs during vasectomy (also adding a layer of safety which is of issue with surgery).

As discussed herein, different aspects of the procedure include length of the procedure, the use of local anesthesia, using a vas-clamp or holding vas superficially to skin with fingers, rate of injection or infusion, needle gauge size and/or length, syringes that can be used, vas deferens characteristics, such as depth of vas from skin, left or right vas, inner and outer diameter of vas, length of vas that is visible, which portion of vas is administration performed, which direction is the administration performed (towards testes or prostate), and ultrasound properties, such as machine specs, probe specs, frequency, intensity, depth, mechanical index, and gain. Such are within the capabilities of a skilled artisan.

The amount of occlusive substance (such as a polymer composition containing a carbon-based nanomaterial) to be administered to a subject can vary based on several different criteria, including the duct, interstitial space, organ, or vessel where it is administered, the size of the lumen, the concentrations of the various components of the substance as administered, the molecular weight of the polymer in the gel, the volume of the gel solution that is administered, and the size of the occlusion that the physician or person who is administering the substance is trying to achieve.

For example, ultrasound imaging of the vas deferens is particularly important for determining the dimensions of its lumen, which determines the amount of substance administered. Administration of an insufficient amount of substance can result in the vas deferens only being partially occluded, while administration of too much substance (particularly at a high rate) can rupture the vas deferens. Thus, according to one embodiment, ultrasound imaging is used to determine the morphometrics and dimensions of the subject's vas deferens, including the inner luminal and outer total diameters, thickness of tunics, and length of the vas deferens. As use of the method increases, a robust database can be generated that will provide valuable information regarding the anatomical and physiological feature of the human male reproductive tract. This new information could potentially further novel advancements in male reproductive health, and will help to standardize known anatomical information on the vas deferens. In 107 men in China, it was reported that the average inner diameter was 0.56 mm and the average outer diameter was 2.17 mm (Liu, X. et al.). However, various other studies have shown that the inner diameter can be as little as 0.31 mm and can dilate as large as 1.8 mm (Liu, X. et al.). The injection volume of the occlusive agent that should be delivered to each individual is a function based on several criteria including: the size of the individual's vas lumen, the concentration of the polymer, the molecular weight of the polymer, monomer ratio of the polymer, injection speed, and desired plug length. Ultrasound allows for the determination of the patient's lumen and plug length. The same paper by Liu et al., reports that the mean rupture volume is around 0.05 mL for 1 cm of vas. During the inventive procedure, ultrasound can be used to ensure that the substance has occluded the lumen and that additional material should not be injected in order to prevent rupturing of the vas.

The carbon-based nanomaterial containing composition can be administered into the as lumen by hand through a standard hypodermic needle and syringe, such as those manufactured by Becton Dickinson (Franklin Lakes, NJ). In one embodiment, an injection device is used for the procedure wherein said device maintains an almost constant injection speed and volume during the injection. In one embodiment, this injection device is a pressure-controlled syringe. The polymeric composition can be provided in a pre-filled syringe, vial, or other suitable container. Alternatively, the vas deferens can be cannulated or catheterized by way of insertion of tubing and a vas-occlusive polymer can be administered mechanically through the use of an infusion pump, such as those manufactured by Cole-Parmer (Vernon Hills, IL). The use of an infusion pump facilitates precise, controlled flow rates and quantities of vas-occlusive polymer solution into the vas deferens lumen.

In embodiments, the device or composition is monitored at various times following administration. For example, it can be monitored using ultrasound at various times, including, days, weeks, months, and years after administration to determine whether it is still there and to monitor its integrity. Monitoring is useful for determining that the composition has polymerized to form a gel, the location of the gel, stability of the gel, effectiveness of the gel, and longevity of the gel, as well as its use as a contraceptive. In addition, ejaculates of the subject can be monitored and sperm counted and measured for viability, motility, activity, etc. and compared to the ultrasound results. Such monitoring can determine the need for a follow-up procedure, such as re-administration of the vas-occlusive polymer to the subject.

In embodiments, the longevity or stability of the polymer gel is estimated by counting the number or concentration of microbubbles inside the gel. The longevity or stability of the polymer gel can be further estimated by determining the echogenicity of the polymer gel using ultrasound. Alternatively, the longevity or stability of the polymer gel can be evaluated by observing the shape, size, or attachment of the polymer gel to the lumen of the vas deferens by way of ultrasound.

In embodiments, solutions of the invention are formulated with microbubbles which incorporate an agent which renders sperm immotile, infertile, or inviable. The agent can be incorporated inside the microbubbles or conjugated to the microbubbles.

Another embodiment of the invention is a method of delivering of an agent to the lumen of the vas deferens. The method includes non-surgically or surgically isolating the vas deferens of a subject, administering a solution into the lumen of the vas deferens, and applying ultrasonic energy at a frequency which is capable of lysing microbubbles present in the solution, thereby releasing the agent into the lumen of the vas deferens. Alternatively, the microbubbles may be allowed to slowly dissolve without the use of ultrasound such that the agent is released to the lumen at a constant rate over time. In this way, the vas-occlusive polymer provides both a physical barrier to the passage of a sperm as well as targeted inhibition of sperm cells.

For example, in one embodiment, focused ultrasound is applied at a particular frequency which causes the microbubbles to vibrate. At a particular threshold of intensity and/or frequency, the microbubbles can be destroyed, which can cause a local shock wave, resulting in cavitation and lysing of the gel. Thus, the use of ultrasound can provide a non-invasive method of reversing the vas-occlusive contraception provided by the invention. Accordingly, one embodiment of the invention provides a method of reversal of a vas-occlusive contraception comprising applying ultrasonic energy to a vas-occlusive gel plug at a frequency and/or intensity that is capable of destroying microbubbles inside the vas-occlusive gel plug, thereby lysing and destroying the occlusion.

In one embodiment, a level of ultrasonic energy needed for microbubble cavitation is determined. For example, a detector transducer receives a scattered level of ultrasonic energy, indicative of stable cavitation. Accordingly, a method for *in vitro* or *ex vivo* testing of microbubble cavitation is used to determine acoustic pressures necessary for reversal. In one aspect, the gel with microbubbles is precipitated in dialysis tubing. By way of example, the gel with microbubbles is precipitated in an excised vas deferens or synthetic vas deferens tissue, and an ultrasound probe is applied at varying frequencies, wherein for each frequency, the amount of gel lost is measured. Once a measurement is recorded which is expected to adequately reverse, de-precipitate, liquefy, dissolve, or flush out the polymer gel, such a frequency can be used to reverse, de-precipitate, liquefy, dissolve, or flush out the polymer occlusion in a subject.

An additional embodiment of the invention includes a method of reversal of a vas-occlusive contraception comprising non-surgically or surgically isolating the vas deferens and administering a solvent or solution in the lumen of the vas deferens that is capable of dissolving a polymer plug disposed in the lumen of the vas deferens. For example, the method of reversal may rely on ultrasonic imaging to determine the location of the vas-occlusive polymer plug in the vas deferens. Then, the vas deferens may be isolated according to the three-finger technique and use of a vas-clamp as previously described. Then, a solvent or solution which is capable of dissolving the polymer may be administered into the lumen of the vas deferens by way of percutaneous injection. Alternatively, the solvent or solution can be used to "flush out" the occlusion. For example, the solvents can include DMSO and the solutions can include sodium or potassium bicarbonate. In one aspect, the solution has a pH from 8-9. As an alternative to bicarbonates, other alkaline solutions can be used. Anywhere from 0.01 - 3 cc of solvent or solution can be injected into the lumen of the vas deferens, such as .01 to .02 cc, .02 to .03 cc, .03 to .04 cc, and so on. However, the rate and volume of injections are limited such that the injection force does not rupture the walls of the vas deferens. The dissolution of the polymer occlusion can then be monitored in real time using ultrasound. Absence of the occlusion and patency of the vas lumen can be confirmed via ultrasound imaging, and ejaculates can be monitored post-procedure to determine restoration of sperm counts in the ejaculate. In this way, successful reversal of contraception can be confirmed.

In some embodiments, reversal of contraception is performed surgically. The vas deferens can be exteriorized, a small slit can be made, and the occlusion may be able to be pulled out (especially in the case of silicone devices). In some embodiments, the occlusion may be mechanically reversed using a miniature drill. If these methods are ineffective, the segment of the vas containing the gel may be ablated and re-anastomosed in a procedure identical to vasovasostomy.

The methods and compositions of the invention can be used to provide long-lasting yet reversible contraception for human males, as well as male animals such as pets, farm animals, zoo animals, and wildlife. The methods have numerous advantages over other forms of contraception such as vasectomy or neutering in terms of reversibility, costs, ease of administration, and lack of complications. Further, as the method involves a one-time administration of a long-lasting contraceptive agent, the method lacks the issues associated with contraceptive drugs or hormones such as adverse effects and lack of compliance.

The methods offer several medical benefits over vasectomy due to the fact that they are significantly less invasive and do not require surgical ablation of the vas deferens. First, a percutaneous injection has been shown to be less painful than incision and exteriorization of the vas as during vasectomy and has less chance for hematoma and infection. Secondly, it is believed that implanting a polymer hydrogel may reduce the chance for granuloma formation; if the pores of a hydrogel allow fluids and small molecules to travel through, this may prevent sperm from extravasating and anti-sperm antibodies may decrease or build up at a slower rate. Thirdly, by allowing fluids to travel through or around the hydrogel, then hydrostatic pressure in the vas deferens will decrease. The buildup of hydrostatic pressure in the vas and epididymis after vasectomy is believed to be a major cause of post-vasectomy pain syndrome. Pain from post-vasectomy pain syndrome is thought to be also caused by sperm granulomas. Altogether, the methods potentially reduce the chance for a patient to develop granulomas, hematomas, pain, or post-vasectomy pain syndrome.

Further, around 6% of men who receive a vasectomy later undergo vasovasostomy (or vasectomy reversal). Vasovasostomy are difficult microsurgery procedures that requires general anesthesia, is expensive, and long (∼3 hours). Research has shown that patients who have a vasectomy reversal >5-10 years after vasectomy decrease their chance for having offspring from 95% to 65% for reasons including the buildup of anti-sperm antibodies. Embodiments of the present invention provide methods of reversal that are similar to the contraceptive methods except instead of a polymer solution being injected into the vas lumen, a different solution is injected percutaneously into the lumen that de-precipitates, dissolves, or liquefies the occlusive substance. The present reversal methods are significantly shorter and easier to perform than vasovasostomy. After reversal is performed, the physician may confirm the procedure was successful based on if the gel is imageable or not on the ultrasound.

In one embodiment, the device is reversed using ultrasound. The ultrasound waves force the microbubbles to oscillate and/or rupture at a certain threshold causing the device to mechanically, physically, and/or chemically breakapart.

In one embodiment, the device is de-precipitated or dissolved with an injectable solution. The solution may contain, but is not limited to, DMSO, bicarbonate (*e*.*g*., sodium bicarbonate, potassium bicarbonate, etc.). The solution may contain disulfides or a reducing agent to selectively dissolve or de-precipitate the polymer by breaking the cross-links.

The following Examples describe particular implementations of the invention. They are intended to further illustrate the invention and should not be used to limit the scope of the invention.

### Example 1: Effect of Styrene Maleic Acid with and without Graphene Nanoplatelets on Sperm Motility and Viability

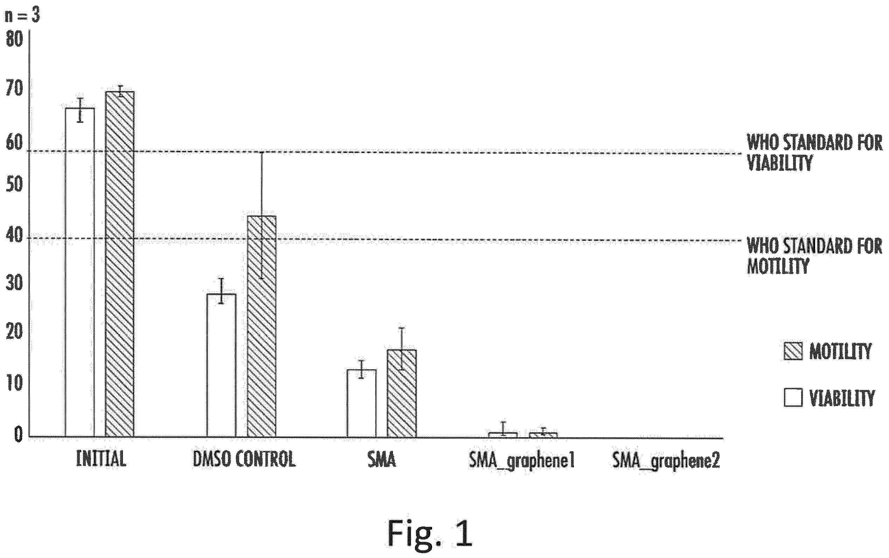

The effect of styrene maleic acid with and without graphene nanoplatelets on sperm motility and viability was studied using an *in vitro* model, with the results shown in FIG. 1. Graphene nanoplatelets functionalized with COOH groups were weighed and dispersed into the styrene maleic acid-DMSO solution at a concentration of 250 µg/ml (SMA_graphenel) and 500 µg/ml (SMA_graphene2). After 5 minutes of interaction time between the sperm and the gels, the sperm on top of the gels had reduced motility and viability. These values (shown in FIG. 1) were significantly below the WHO's standards for viable and motile sperm. Therefore, according to this example, the addition of graphene enhances spermicidal properties to the point where all sperm are rendered infertile by 500 µg/ml graphene concentration.

### Example 2: Methods of Graphene Dispersion and Visualization of the Gel Surface

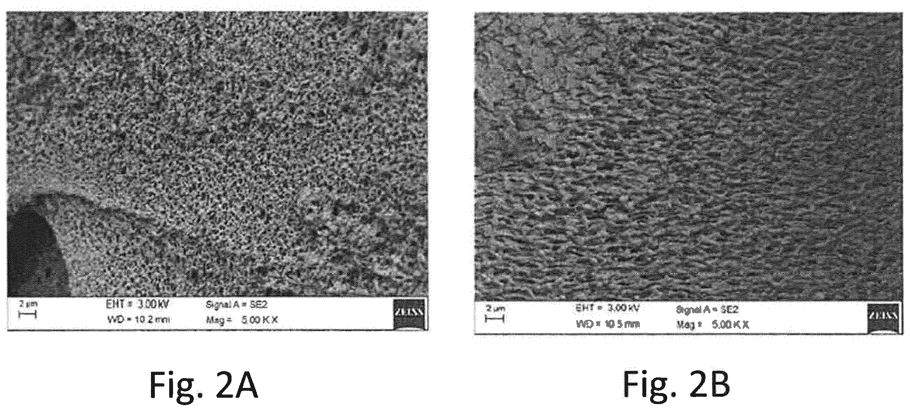

FIGS. 2A and 2B, taken by scanning electron microscopy, display the ultrasonication method (FIG. 2A) versus vortex method (FIG. 2B) for dispersal of graphene in the polymer. As shown in the figures, the graphene is much more uniformly dispersed when the polymer is subjected to ultrasonication. This is important because the agglomeration of graphene is detrimental in terms of cytotoxicity. As a result, ultrasonication is the preferred method for graphene dispersal to enhance biocompatibility. It can also be noted that the surface of the ultrasonication-dispersed graphene has much more uniform pores on its surface.

### Example 3: Measuring Hardness and Elasticity of SMA-Graphene Hydrogel

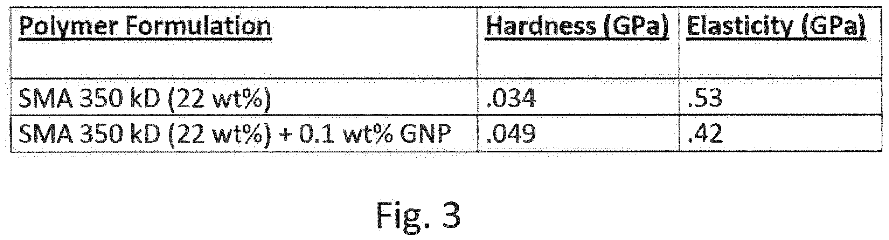

Nanoindentation was performed on polymer gels comprising styrene maleic acid at a molecular weight of 350,000 daltons and 22 wt% with and without the addition of .1 1 wt% graphene nanoplatelets. For each polymer gel, 5 measurements were taken and averaged (shown in the table in FIG. 3). The hardness of the styrene maleic acid (SMA) gel increased by 44.12% and the elasticity decreased by 20.75% with the addition of graphene.

### Example 4: Effect of Graphene Nanoplatelets on Viscosity

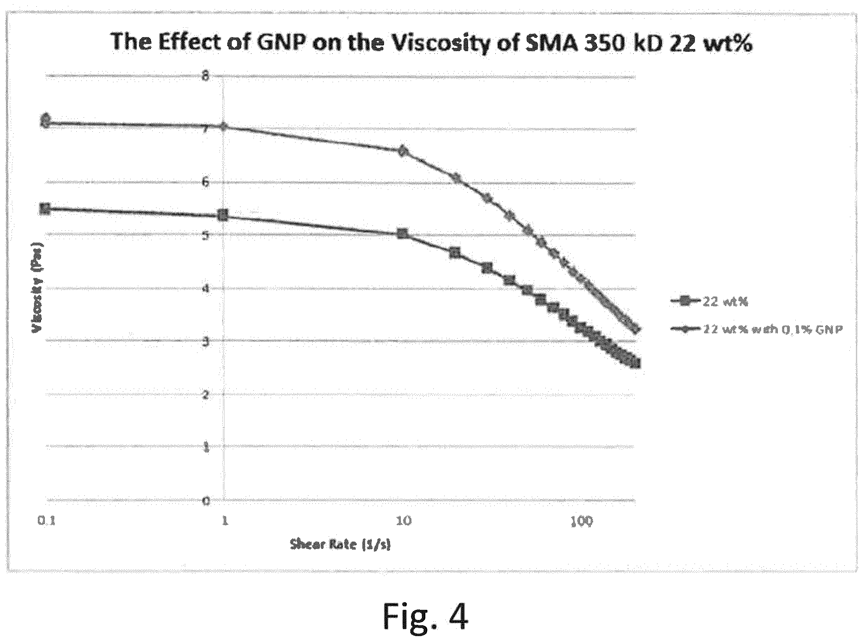

An Anton Paar rheometer (Physica MCR 301) with 49.966 mm and 1.0101° cone-plate geometry was used to measure the viscosity of the following formulations: styrene maleic acid (MW- 350,000) dissolved in DMSO at 22 wt%, and styrene maleic acid (MW-350,000) dissolved in DMSO at 22% with the addition of .1 wt% of graphene nanoplatelets (GNP). The graphene nanoplatelets were dispersed in the solution via ultrasonication. As shown in the graph in FIG. 4, the viscosity of the solution increased from 5.875 Pas to 7.355 Pas with the addition of 0.1 wt% graphene, an overall increase of 25%.

### Example 5: Electrospun Polycaprolactone-Graphene Fiber and Mesh

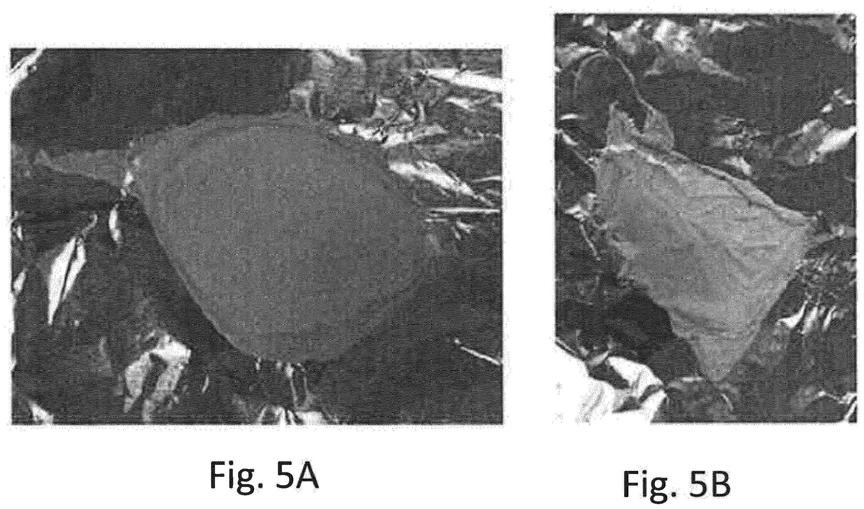

Polycaprolactone (PCL) was dissolved at 12.5 wt% in a 50:50, 70:30, and 90:10 chloroform: DMSO solution and electrospun using a custom rig. It was discovered that the 50:50 mixture was optimal for electrospinning the fibers (FIG. 5A). A mesh was also Electrospun with PCL and the addition of graphene nanoplatelets (FIG. 5B). While it took longer, the graphene-PCL mesh appears more durable and elastic.

### Example 6: Carbon allotropes minimally interacting with materials

Hydrogels may be formulated by suspending carbon allotropes, such as but not limited to single walled carbon nanotubes, multiple walled carbon nanotubes, fullerenes, and graphene ribbons in a solvent. The carbon allotropes can be pre-modified with functional groups or molecules or macromolecules for binding molecules/macromolecules, drug delivery, and/or enhancing the material properties. Additionally, the carbon allotropes can be functionalized or modified at any point during the formation of the final material. Finally, the final material can be designed such that it can change and/or is modified over time to create a dynamic final formulation. This suspension can then be added to a monomer solution that is then polymerized to create a hydrogel that encapsulates the carbon allotropes. It is also possible to suspend the carbon allotropes in the monomer solution and polymerize. It is also possible to add suspended carbon allotropes to or suspend carbon allotropes in polymers that then form hydrogels and/or interpenetrating polymer networks through covalent or non-covalent interactions. In this example the carbon allotropes have minimal interaction with the monomers or polymers and the polymerization or crosslinking/hydrogel forming reaction.

### Example 7: Carbon allotropes interacting directly with materials

Hydrogels may be formulated by suspending carbon allotropes, such as but not limited to single walled carbon nanotubes, multiple walled carbon nanotubes, fullerenes, and graphene ribbons in a solvent. The carbon allotropes can be pre-modified with functional groups or molecules or macromolecules for binding molecules/macromolecules, drug delivery, and/or enhancing the material properties. Additionally, the carbon allotropes can be functionalized or modified at any point during the formation of the final material. Finally, the final material can be designed such that it can change and/or is modified over time to create a dynamic final formulation. This suspension can then be added to a monomer solution that is then polymerized to create a hydrogel that encapsulates the carbon allotropes. It is also possible to suspend the carbon allotropes in the monomer solution and polymerize. It is also possible to add suspended carbon allotropes to or suspend carbon allotropes in polymers that then form hydrogels and/or interpenetrating polymer networks through covalent or non-covalent interactions. In this example the carbon allotropes have direct interaction with the monomers or the polymers but not the polymerization reaction or hydrogel forming mechanism. This interaction can occur through covalent or non-covalent means and the carbon allotropes can be naked or chemically functionalized.

### Example 8: Carbon allotropes having no interaction with materials

Hydrogels may be formulated by suspending carbon allotropes, such as but not limited to single walled carbon nanotubes, multiple walled carbon nanotubes, fullerenes, and graphene ribbons in a solvent. The carbon allotropes can be pre-modified with functional groups or molecules or macromolecules for binding molecules/macromolecules, drug delivery, and/or enhancing the material properties. Additionally, the carbon allotropes can be functionalized or modified at any point during the formation of the final material. Finally, the final material can be designed such that it can change and/or is modified over time to create a dynamic final formulation. This suspension can then be added to a monomer solution that is then polymerized to create a hydrogel that encapsulates the carbon allotropes. It is also possible to suspend the carbon allotropes in the monomer solution and polymerize. It is also possible to add suspended carbon allotropes to or suspend carbon allotropes in polymers that then form hydrogels and/or interpenetrating polymer networks through covalent or non-covalent interactions. In this example the carbon allotropes have no direct interaction with the monomers or the polymers and the polymerization reaction or hydrogel forming mechanism. This interaction can occur through covalent or non-covalent means and the carbon allotropes can be naked or chemically functionalized.

### Example 9: Functionalization of carbon allotropes to enhance biological properties

This technology can be used to add chemicals, molecules, and/or biomolecules to the carbon allotropes that are then passively (no covalent or no non-covalent interactions with the monomers or polymers) or actively (covalent or non-covalently interacting with the monomers or the polymers). For example, sperm binding antibody or antibodies (or other biomolecules) may be covalently or non-covalently bound to a carbon allotrope. Then these sperm-binding carbon allotropes can then be passively or actively encapsulated within a hydrogel. The benefits of this material might be such that it improves the sperm-blocking or binding of the material or implant which would be useful for male or female contraception. Additionally, by adding the carbon allotrope to the hydrogel additional, beneficial properties are conferred like increase in hardness and durability, which can be important properties for implants.

The present disclosure has been described with reference to particular embodiments having various features. In light of the disclosure provided above, it will be apparent to those skilled in the art that various modifications and variations can be made in the practice of the present invention without departing from the scope or spirit of the invention. One skilled in the art will recognize that the disclosed features may be used singularly, in any combination, or omitted based on the requirements and specifications of a given application or design. When an embodiment refers to "comprising" certain features, it is to be understood that the embodiments can alternatively "consist of" or "consist essentially of" any one or more of the features. Other embodiments of the invention will be apparent to those skilled in the art from consideration of the specification and practice of the invention. It is noted in particular that where a range of values is provided in this specification, each value between the upper and lower limits of that range is also specifically disclosed.

The upper and lower limits of these smaller ranges may independently be included or excluded in the range as well. The singular forms "a," "an," and "the" include plural referents unless the context clearly dictates otherwise. It is intended that the specification and examples be considered as exemplary in nature and that variations that do not depart from the essence of the invention fall within the scope of the invention. Further, all of the references cited in this disclosure are each individually incorporated by reference herein in their entireties and as such are intended to provide an efficient way of supplementing the enabling disclosure of this invention as well as provide background detailing the level of ordinary skill in the art.

## Claims

1. A composition comprising:
a carbon-based nanomaterial or carbon allotrope, a polymer or network forming agent, and a solvent,
wherein the carbon-based nanomaterial or carbon allotrope is present in the composition at a concentration which enhances the efficacy of the composition as an occlusive agent upon administration into a body lumen.

2. The composition of claim 1, wherein the composition is a hydrogel or forms a hydrogel *in situ* upon administration into a body lumen.

3. The composition of claim 1 or 2, wherein the carbon-based nanomaterial or carbon allotrope is present in the composition at a concentration which enhances the efficacy of the composition as a contraceptive agent, or at a concentration that decreases the fertility, motility, and/or viability of sperm cells, or at a concentration that increases the hardness and/or durability of the hydrogel, or at a concentration that improves the mechanical properties of the hydrogel, or at a concentration which alters the viscosity of the hydrogel.

4. The composition of any one of claims 1 to 3, wherein the carbon-based nanomaterial or carbon allotrope is conjugated with a ligand comprising a small molecule, a protein, a peptide, an antibody, a nucleic acid, or fragment thereof, an aptamer, DNA, RNA, PNA, an enzyme, a sugar, a polysaccharide, a small molecule, a large molecule, a polymer, or a combination thereof.

5. The composition of any one of claims 1 to 4, wherein the carbon-based nanomaterial or carbon allotrope conjugation is performed passively through adsorption with or without post-chemical activation of the carbon-based nanomaterial or carbon allotrope, actively through covalent bonding, or through placement of the ligand actively or passively to allow another molecule of interest to bind.

6. The composition of any one of claims 1 to 5, wherein the carbon-based nanomaterial or carbon allotrope is functionalized with carboxylic acid (COOH) or a carboxylic group, amine (NH2), ammonia (NH3) or ammonium, pristine, argon (Ar), silicon (Si), a fluorocarbon, nitrogen (N2), fluorine (F), oxygen, alkyl, cycloalkyl, aryl, alkylaryl, amide, ester, ether, sulfonamide, carboxylate, sulfonate, phosphonate, fluorocarbons, carbonates, nitro, halogens (bromine, chlorine, fluorine), boron, boronic acids, biomacromolecules including sugars and proteins, a polymer, and supramolecular/coordination complexes including metal coordination complexes, and supramolecular complexes.

7. The composition of any one of claims 1 to 6, wherein the carbon-based nanomaterial or carbon allotrope comprises one or more of graphene, graphene powder, graphene oxide, nanoscale graphene oxide, reduced graphene oxide, nanoscale graphene oxide, graphene nanoribbons, graphene nanotubes, graphene sheets, graphene films, granulated graphene, graphene quantum dots, graphene nanoribbons, graphene nanocoils, graphene aerogels, graphene nanoplatelets, carbon nanotubes, carbon nanosheets, carbon nanocones, carbon nanoribbons, buckyballs, and/or fullerenes.

8. The composition of any one of claims 1 to 7, wherein the carbon-based nanomaterial or carbon allotrope comprises graphene.

9. The composition of any one of claims 1 to 8, wherein the polymer comprises one or more of styrene maleic anhydride, styrene maleic acid, ethylene vinyl alcohol (EVOH), ethylene vinyl acetate, poly(ethylene glycol) (PEG), poly-L-lactic acid (PLLA), poly(lactic-co-glycolic acid) (PLGA), poly lactide (PLA), poly(glycolic acid) (PGA), poly(vinyl alcohol) (PVA), polydimethylsiloxane (PDMS), poly(isopropylacrylate) (PIPA), poly(ethylene-vinyl acetate) (PEVA), PEG styrene, any block copolymer, poly(styrene)-block-poly(ethylene glycol), a nylon polymer, Teflon RFE, polyetherketone etherketone ketone (PEKEKK), fluorinated high density polyethylene (FLPE), neoprene, (PETE), Teflon FEP, Teflon PFA, polymethylpentene (PMP), methyl palmitate, poly(N-isopropylacrylamide) (NIPA), polycarbonate, polyethersulfone, polycaprolactone, polymethyl methacrylate, polypropylene, polyurethane, polystyrene, polyisobutylene, nitrocellulose, medical grade silicon, cellulose acetate butyrate, polyacrylonitrile, poly(lactide-co-caprolactone) (PLCL), chitosan, alginate, polymethyl methacrylate, polyacrylonitrile, poly (carbonate-urethane), poly (vinylacetate), nitrocellulose, cellulose acetate, urethane, urethane/carbonate, polylactic acid, polyacrylamide (PAAM), poly (N-isopropylacrylamine) (PNIPAM), poly (vinylmethylether), poly (ethylene oxide), poly (ethyl (hydroxyethyl) cellulose), poly(2-ethyl oxazoline), poly(e-caprolactone), polydiaoxanone, polyanhydride, trimethylene carbonate, poly(β-hydroxybutyrate), poly(g-ethyl glutamate), poly(DTH-iminocarbonate), poly(bisphenol A iminocarbonate), poly(orthoester) (POE), polycyanoacrylate (PCA), polyphosphazene, polyethyleneoxide (PEO), polyacrylic acid (PAA), polyacrylonitrile (PAN), polyvinylpyrrolidone (PVP), polyglycolic lactic acid (PGLA), poly(2-hydroxypropyl methacrylamide) (pHPMAm), poly(vinyl alcohol) (PVOH), PEG diacrylate (PEGDA), poly(hydroxyethyl methacrylate) (pHEMA), N-isopropylacrylamide (NIPA), poly(vinyl alcohol) poly(acrylic acid) (PVOH-PAA), collagen, silk, fibrin, gelatin, hyaluron, cellulose, chitin, dextran, casein, albumin, ovalbumin, heparin sulfate, starch, agar, heparin, fibronectin, fibrin, keratin, pectin, and/or elastin.

10. The composition of any one of claims 1 to 9, wherein the solvent comprises a buffered aqueous solution, dimethyl sulfoxide, ethyl acetate, methanol, acetone, acetonitrile, tetrahydrofuran (THF), dioxane, dimethylformamide (DMF), heptane, hexane, pentane, petroleum ether, benzene, toluene, o-xylene, m-xylene, p-xylene, ethanol, methanol, t-butyl alcohol, diethylene glycol, glycerol, ethylene glycol, chloroform, dichloromethane, 1,2-dichloroethane, hexafluoroisopropanol, a deuterated solvent, or a fluorocarbon solvent.

11. The composition of any one of claims 1 to 10, wherein the hydrogel includes contrast agents, imaging agents, therapeutic drugs, antimicrobials, anti-inflammatories, spermicidal agents, vasodilators, steroids, hormones, ionic solutions, proteins, nucleic acids, antibodies, or fragments thereof.

12. Composition comprising a carbon-based nanomaterial or carbon allotrope, a polymer, and a solvent for use in a method of occluding a body lumen comprising:
administering the composition into the body lumen, wherein the carbon-based nanomaterial or carbon allotrope is present in the composition at a concentration which enhances the efficacy of the composition as an occlusive agent upon administration into a body lumen; and
polymerizing the composition or forming a mass from the composition in the body lumen.

13. Composition for use according to claim 12, wherein the body lumen is an artery, a vein, a vas deferens, a fallopian tube, a uterus, a duct, interstitial space, or an organ.

14. Composition for use according to claim 12 or 13, wherein the carbon-based nanomaterial or carbon allotrope comprises one or more of graphene, graphene powder, graphene oxide, nanoscale graphene oxide, reduced graphene oxide, nanoscale graphene oxide, graphene nanoribbons, graphene nanotubes, graphene sheets, graphene films, granulated graphene, graphene quantum dots, graphene nanoribbons, graphene nanocoils, graphene aerogels, graphene nanoplatelets, carbon nanotubes, carbon nanosheets, carbon nanocones, carbon nanoribbons, buckyballs, and/or fullerenes.

15. Use of the composition of any one of claims 1 to 11 to provide contraception.
